# EUROPEAN PATENT SPECIFICATION

(11) **EP 1 007 098 B1**
(45) Date of publication and mention of the grant of the patent: **09.08.2006**
(21) Application number: 98911615.7
(22) Date of filing: 12.03.1998
(51) Int. Cl.: A61K 48/00, A61K 31/70, A61K 9/00

(54) **DOWN-REGULATION OF GENE EXPRESSION BY COLORECTAL ADMINISTRATION OF SYNTHETIC OLIGONUCLEOTIDES**
VERMINDERUNGSREGULATION DER GENEXPRESSION DURCH KOLOREKTALE VERABREICHUNG VON SYNTHETISCHEN OLIGONUKLEOTIDEN
RETRO-REGULATION D'EXPRESSION GENIQUE PAR ADMINISTRATION COLO-RECTALE D'OLIGONUCLEOTIDES SYNTHETIQUES

(30) Priority: 12.03.1997 US 40738 P; 30.04.1997 US 846417
(43) Date of publication of application: 14.06.2000
(73) Proprietor: IDERA PHARMACEUTICALS, INC., Cambridge, MA 02139 (US)
(72) Inventor: ZHANG, Ruiwen, Birmingham, AL 35244 (US); AGRAWAL, Sudhir, Shrewsbury, MA 01545 (US)
(74) Representative: Grund, Martin
(86) International application number: PCT/US1998/004914
(87) International publication number: WO 1998/040058

(56) References cited:
- WO-A-94/17093
- WO-A-96/12497
- WO-A-96/18390
- WO-A-97/05267
- AGRAWAL S: "Antisense oligonucleotides: towards clinical trials" TRENDS IN BIOTECHNOLOGY, vol. 14, no. 10, October 1996, page 376-387 XP004035728
- PATERSON B M ET AL: "STRUCTURAL GENE IDENTIFICATION AND MAPPING BY DNA.MRNA HYBRID-ARRESTED CELL-FREE TRANSLATION" PROCEEDINGS OF THE NATIONAL ACADEMY OF SCIENCES OF THE USA, vol. 74, no. 10, 1 October 1977, pages 4370-4374, XP000575587 cited in the application
- AGRAWAL S ET AL: "OLIGODEOXYNUCLEOSIDE PHOSPHORAMIDATES AND PHOSPHOROTHIOATES AS INHIBITORS OF HUMAN IMMUNODEFICIENCY VIRUS" PROCEEDINGS OF THE NATIONAL ACADEMY OF SCIENCES OF USA, vol. 85, 1 October 1988, pages 7079-7083, XP000574956 cited in the application
- SUDHIR AGRAWAL: "ANTISENSE OLIGONUCLEOTIDES AS ANTIVIRAL AGENTS" TRENDS IN BIOTECHNOLOGY, vol. 10, no. 5, 1 May 1992, pages 152-158, XP000272382 cited in the application
- AGRAWAL S ET AL: "INHIBITION OF HUMAN IMMUNODEFICIENCY VIRUS IN EARLY INFECTED AND CHRONICALLY INFECTED CELLS BY ANTISENSE OLIGODEOXYNUCLEOTIDES AND THEIR PHOSPHOROTHIOATE ANALOGUES" PROCEEDINGS OF THE NATIONAL ACADEMY OF SCIENCES OF USA, vol. 86, no. 20, 1 October 1989, pages 7790-7794, XP000070876 cited in the application
- STEPHENSON M L ET AL: "INHIBITION OF ROUS SARCOMA VIRAL RNA TRANSLATION BY A SPECIFIC OLIGODEOXYRIBONUCLEOTIDE" PROCEEDINGS OF THE NATIONAL ACADEMY OF SCIENCES OF THE USA, vol. 75, no. 1, 1 January 1978, pages 285-288, XP000575586 cited in the application

### SEQUENCE LISTING

(1) GENERAL INFORMATION:
   (i) APPLICANT: IDERA PHARMACEUTICALS, INC.
   (ii) TITLE OF INVENTION: DOWN-REGULARION OF GENE EXPRESSION BY COLORECTAL ADMINISTRATION OF SYNTHETIC OLIOGNUCLEOTIDES
   (iii) NUMBER OF SEQUENCES: 21
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (v) CURRENT APPLICATION DATA:
      (A) APPLICATION NUMBER: EP 98 911 615.7
      (B) FILING DATE: 12-MAR-1998
      (C) CLASSIFICATION:
   (vi) PRIOR APPLICATION DATA:
      (A) APPLICATION NUMBER: US 08/846,417
      (B) FILING DATE: 30-APR-1997
   (vii) PRIOR APPLICATION DATA:
      (A) APPLICATION NUMBER: US 60/040,738
      (B) FILING DATE: 12-MAR-1997
(2) INFORMATION FOR SEQ ID NO:1:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:1:
      CTCTCGCACC CATCTCTCTC CTTCU 25
(2) INFORMATION FOR SEQ ID NO:2:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:2:
      CTCTCGCACC CATCTCTCTC CTUCU 25
(2) INFORMATION FOR SEQ ID NO:3:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO

   (xi) SEQUENCE DESCRIPTION: SEQ ID NO:3:
   CTCTCGCACC CATCTCTCTC CUUCU 25
(2) INFORMATION FOR SEQ ID NO:4:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:4:
      CTCTCGCACC CATCTCUCUC CUUCU 25
(2) INFORMATION FOR SEQ ID NO:5:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:5:
      CTCTCGCACC CAUCUCUCUC CUUCU 25
(2) INFORMATION FOR SEQ ID NO:6:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:6:
      CUCUCGCACC CAUCUCUCUC CUUCU 25
(2) INFORMATION FOR SEQ ID NO:7:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:7:
      CTCTCGCACC CATCTCTCTC CTTCU 25
(2) INFORMATION FOR SEQ ID NO:8:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:8:
      CUCUCGCACC CATCTCTCTC CUUCU 25
(2) INFORMATION FOR SEQ ID NO:9:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:9:
      CUCUCGCACC CATCTCUCUC CUUCU 25
(2) INFORMATION FOR SEQ ID NO:10:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:10:
      CUCUCGCACC CAUCUCUCUC CUUCU 25
(2) INFORMATION FOR SEQ ID NO:11:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:11:
      CUCUCGCACC CATCTCTCUC CUUCU 25
(2) INFORMATION FOR SEQ ID NO:12:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:12:
      CUCUCGCACC CAUCTCTCTC CUUCU 25
(2) INFORMATION FOR SEQ ID NO:13:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:13:
      CUCUCGCACC CATCTCTCTC CUUCU 25
(2) INFORMATION FOR SEQ ID NO:14:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:14:
      CUCTCGCACC CAUCUCUCUC CUUCU 25
(2) INFORMATION FOR SEQ ID NO:15:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:15:
      CUCTCGCACC CATCTCTCUC CUUCU 25
(2) INFORMATION FOR SEQ ID NO:16:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:16:
      CTCTCGCACC CATCTCTCTC CTTC 24
(2) INFORMATION FOR SEQ ID NO:17:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:17:
      CTCTCGCACC CATCTCTCTC CTTCT 25
(2) INFORMATION FOR SEQ ID NO:18:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:18:
      CUCTCGCACC CATCTCTCTC CTTCT 25
(2) INFORMATION FOR SEQ ID NO:19:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:19:
      CUCUCGCACC CATCTCTCTC CTTCT 25
(2) INFORMATION FOR SEQ ID NO:20:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:20:
      CUCUCGCACC CAUCUCTCTC CTTCT 25
(2) INFORMATION FOR SEQ ID NO:21:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (xi) SEQUENCE DESCRIPTION: SEQ ID NO:21:
      CUCUCGCACC CAUCUCUCUC CTTCT 25

## Description

### BACKGROUND OF THE INVENTION

The present invention relates to the control of gene expression. More particularly, this invention relates to the use of synthetic oligonucleotides to down-regulate the expression of a gene in an animal.

The potential for the development of an antisense oligonucleotide therapeutic approach was first suggested in three articles published in 1977 and 1978. Paterson et al. (*Proc. Natl. Acad. Sci. (USA)* (1977) 74:4370-4374) discloses that cell-free translation of mRNA can be inhibited by the binding of an oligonucleotide complementary to the mRNA. Zamecnik et al. (*Proc. Natl. Acad. Sci. (USA)* (1978) 75:280-284 and 285-288) discloses that a 13mer synthetic oligonucleotide that is complementary to a part of the Rous sarcoma virus (RSV) genome inhibits RSV replication in infected chicken fibroblasts and inhibits RSV-mediated transformation of primary chick fibroblasts into malignant sarcoma cells.

These early indications that synthetic oligonucleotides can be used to inhibit virus propagation and neoplasia have been followed by the use of synthetic oligonucleotides to inhibit a wide variety of viruses, such as HIV (see, e.g., U.S. Patent No. 4,806,463); influenza (see, e.g., Leiter et al. (1990) (*Proc. Natl. Acacl. Sci. (USA)* 87:3430-3434); vesicular stomatitis virus (see, e.g., Agris et al. (1986) *Biochem*. 25:6268-6275); herpes simplex (see, e.g., Gao et al. (1990) *Antimicrob. Agents Chem.* 34:808-812); SV40 (see, e.g., Birg et al. (1990) *(Nucleic Acids Res.* 18:2901-2908); and human papilloma virus (see, e.g., Storey et al. (1991) *(Nucleic Acids Res.* 19:4109-4114). The use of synthetic oligonucleotides and their analogs as antiviral agents has recently been extensively reviewed by Agrawal *(Trends in Biotech.* (1992) 10:152-158).

In addition, synthetic oligonucleotides have been used to inhibit a variety of non-viral pathogens, as well as to selectively inhibit the expression of certain cellular genes. Thus, the utility of synthetic oligonucleotides as agents to inhibit virus propagation, propagation of non-viral, pathogens and selective expression of cellular genes has been well established.

Improved oligonucleotides have more recently been developed that have greater efficacy in inhibiting such viruses, pathogens and selective gene expression. Some of these oligonucleotides having modifications in their internucleotide linkages have been shown to be more effective than their unmodified counterparts. For example, Agrawal et al. (*Proc. Natl. Acad. Sci. (USA)* (1988) 85:7079-7083) teaches that oligonucleotide phosphorothioates and certain oligonucleotide phosphoramidates are more effective at inhibiting HIV-1 than conventional phosphodiester-linked oligodeoxynucleotides. Agrawal et al. (*Proc. Natl. Acad. Sci. (USA)* (1989) 86:7790-7794) discloses the advantage of oligonucleotide phosphorothioates in inhibiting HIV-1 in early and chronically infected cells.

In addition, chimeric oligonucleotides having more than one type of internucleotide linkage within the oligonucleotide have been developed. Pederson et al. (U.S. Patent Nos. 5,149,797 and 5,220,007 discloses chimeric oligonucleotides having an oligonucleotide phosphodiester or oligonucleotide phosphorothioate core sequence flanked by nucleotide methylphosphonates or phosphoramidates. Furdon et al. (*Nucleic Acids Res*. (1989) 17:9193-9204) discloses chimeric oligonucleotides having regions of oligonucleotide phosphodiesters in addition to either oligonucleotide phosphorothioate or methylphosphonate regions. Quartin et al. (*Nucleic Acids Res.* (1989) 17:7523-7562) discloses chimeric oligonucleotides having regions of oligonucleotide phosphodiesters and oligonucleotide methylphosphonates. Inoue et al. *(FEBS Lett.* (1987) 215:237-250) discloses chimeric oligonucleotides having regions of deoxyribonucleotides and 2'-O-methyl-ribonucleotides.

Many of these modified oligonucleotides have contributed to improving the potential efficacy of the antisense oligonucleotide therapeutic approach. However, certain deficiencies remain in the known oligonucleotides, and these deficiencies can limit the effectiveness of such oligonucleotides as therapeutic agents. For example, Wickstrom (*J. Biochem. Biophys. Meth.* (1986) 13:97-102) teaches that oligonucleotide phosphodiesters are susceptible to nuclease-mediated degradation, thereby limiting their bioavailability *in vivo.* Agrawal et al. *(Proc. Natl. Acad. Sci. (USA)* (1990) 87:1401-1405) teaches that oligonucleotide phosphoramidates or methylphosphonates when hybridized to RNA do not activate RNase H, the activation of which can be important to the function of antisense oligonucleotides. Thus, a need for methods of controlling gene expression exists which uses oligonucleotides with improved therapeutic characteristics.

Several reports have been published on the development of phosphorothioate-linked oligonucleotides as potential anti-AIDS therapeutic agents. Although extensive studies on chemical and molecular mechanisms of oligonucleotides have demonstrated the potential value of this novel therapeutic strategy, little is known about the pharmacokinetics and metabolism of these compounds *in vivo*.

Several preliminary studies on this topic have been published. Agrawal et al. (*Proc. Natl. Acad. Sci*. *(USA)* (1991) 88:7595-7599) describes the intravenously and intraperitoneally administration to mice of a 20mer phosphorothioate linked-oligonucleotide. In this study, approximately 30% of the administered dose was excreted in the urine over the first 24 hours with accumulation preferentially in the liver and kidney. Plasma half-lives ranged from about 1 hour t_{1/2α}) and 40 hours (t_{1/2β}), respectively. Similar results have been reported in subsequent studies (Iversen (1991) *Anti-Cancer Drug Design* 6:531-538; Iversen (1994) *Antisense Res. Devel*. 4:43-52; and Sands (1994) *Mol. Pharm*. 45:932-943). However, stability problems may exist when oligonucleotides are administered intravenously and intraperitoneally. More recent studies have demonstrated that two hybrid oligonucleotides which are two end-protected [³⁵S]-radiolabelled analogs of a 25mer oligonucleotide phosphorothioate, one containing segments of 2 - 'O-methyloligoribonucleotide phosphorothioates at both 3'- and 5'-termini (MBO 1) and another containing methyl phosphonate linkages at both 3'-and 5'-termini (MBO 2) exhibited enterohepatic circulation in rats after i.v. bolus administration, with a significantly better *in vivo* stability than the oligonucleotide phosphorothioate (Zhang et al. (1995) *Biochem. Pharmacol.* 49:929-939; Zhang et al. (1995) *Biochem. Pharmacol.* 50:571-576; and Zhang et al. (1996) *J. Pharm. Exp. Ther.* 278:971-979). Hybrid oligonucleotides have also been administered orally to rats with little degradation (Zhang et al. (1995) *Biochem. Pharm.* 50:545-556).

WO 94/17093 discloses an oligonucleotide analog comprising at least one ribonucleotide alkylphosphonate or alkylphosphonothioate. This analog is preferably from 2 to 60 nucleotides in length and has at least one ribonucleotide substituted at the 2' position of its ribose group. Also disclosed are therapeutic formulations comprising this oligonucleotide analog, methods of inhibiting the expression of a gene from a virus, pathogenic organism, or cell, the expression of which is associated with a disease state, and methods of treating a mammal infected with a virus or pathogenic organism or afflicted with a disorder resulting from the expression of a cellular gene.

WO 96/12497 discloses a method of down-regulating the expression of a gene in an animal, wherein a pharmalogical formulation comprising an oligonucleotide complementary to the gene is orally administered to an animal. The oligonucleotide administered has non-phosphodiester internucleotide linkages and includes at least one 2'-substituted ribonucleotide, the oligonucleotide inhibiting the expression of a product of the gene, thereby down-regulating the expression of the gene.

WO 97/05267 discloses a composition for delivery of a polynucleotide to mucosal, neural, or other cells, comprising a GN1-binding protein and a polynucleotide in association with the binding protein; and a method for modulating immunity comprising administering the composition to an animal and expressing the polynucleotide whereby the animal generates an immune response to the product of the polynucleotide. WO 97/05267 also discloses a method for gene therapy comprising administering to an animal a GM1-binding protein and a functional polynucleotide and expressing the polynucleotide in the animal whereby the function of the polynucleotide confers on the animal a therapeutic effect.

However, there still remains a need to develop more effective therapeutic methods of down-regulating the expression of genes which can be easily manipulated to fit the animal and condition to be treated, and the gene to be targeted.

Preferably, these methods should be simple, painless, and precise in effecting the target gene.

### SUMMARY OF THE INVENTION

The present invention provides a pharmaceutical formulation comprising an oligonucleotide of about 12 to about 50 nucleotides having non-phosphodiester internucleotide linkages and being complementary to a targeted gene, for colorectal administration to a mammal, wherein the oligonucleotide has 3' and 5' termini and further comprises at least one 2'-substituted ribonucleotide at the 5' terminus, at the 3' terminus, or at the 5' terminus and the 3' terminus.

In a further aspect the present invention provides the use of an oligonucleotide of about 15 to 25 nucleotides and comprising non-phosphodiester internucleotide linkages for the manufacture of a pharmaceutical formulation for colorectal administration for the treatment of virus infections, infections by pathogenic organisms, or diseases or disorders resulting from abnormal gene expression or from the expression of an abnormal gene product wherein the oligonucleotide has 3' and 5' termini and further comprises at least one 2'-substituted ribonucleotide at the 5' terminus, at the 3' terminus, or at the 5' terminus and the 3' terminus.

It has been discovered that certain end-modified oligonucleotides are relatively stable *in vivo* following colorectal administration to an animal, and that these molecules are successfully absorbed from the intestinal tract and distributed to various body tissues with little degradation. Thus, this form of administration bypasses the complications which may be experienced during oral, intravenous and other modes of *in vivo* administration. This discovery has been exploited to develop the present invention, which is a method of down-regulating the expression of a gene in an animal.

This method is also a means of examining the function of various genes in an animal, including those essential to animal development. Presently, gene function can only be examined by the arduous task of making a "knock out" animal such as a mouse. This task is difficult, time-consuming and cannot be accomplished for genes essential to animal development since the "knock out" would produce a lethal phenotype. The present invention overcomes the shortcomings of this model.

In the use of the invention, a pharmaceutical formulation containing an oligonucleotide complementary to the targeted gene is colorectally administered in a pharmaceutically acceptable carrier to the animal harboring the gene. The oligonucleotide inhibits the expression of the gene, thereby down-regulating its expression.

For purposes of the invention, the term "animal" is meant to encompass humans as well as other mammals, as well as reptiles amphibians, and insects. The term "colorectal administration" or "rectal administration" or "colorectally administered" refers to the provision of the pharmaceutical formulation of the invention to any part of the large intestine via surgical implantation, anal administration, or any other mode of placement therein.

The oligonucleotide being administered has non-phosphodiester linkages. As used herein, the term "oligonucleotide" is meant to include polymers of two or more nucleotides or nucleotide analogs connected together via 5' to 3' internucleotide linkages which may include any linkages that are known in the antisense art. Such molecules have a 3' terminus and a 5' terminus.

The term "non-phosphodiester-linked oligonucleotide" as used herein is an oligonucleotide in which all of its nucleotides are covalently linked via a synthetic linkage, i.e., a linkage other than a phosphodiester between the 5' end of one nucleotide and the 3' end of another nucleotide in which the 5' nucleotide phosphate has been replaced with any number of chemical groups. Preferable synthetic linkages include alkylphosphonates, phosphorothioates, phosphorodithioates, alkylphosphonothioates, phosphoramidates, phosphoramidites, phosphate esters, carbamates, carbonates, phosphate triesters, acetamidate, and carboxymethyl esters. In one embodiment of the invention, the all of the nucleotides of the oligonucleotide comprises are linked via phosphorothioate and/or phosphorodithioate linkages, and in one particular embodiment, the nucleotides are all phosphorothioate linked.

In some embodiments of the invention, the oligonucleotides administered are further modified. As used herein, the term "modified oligonucleotide" encompasses oligonucleotides with modified nucleic acid(s), base(s), and/or sugar(s) other than those found in nature. For example, a 3', 5'-substituted oligonucleotide is an oligonucleotide having a sugar which, at both its 3' and 5' positions is attached to a chemical group other than a hydroxyl group (at its 3' position) and other than a phosphate group (at its 5' position).

A modified oligonucleotide may also be one with added substituents such as diamines, cholestryl, or other lipophilic groups, or a capped species. In addition, unoxidized or partially oxidized oligonucleotides having a substitution in one nonbridging oxygen per nucleotide in the molecule are also considered to be modified oligonucleotides. Also considered as modified oligonucleotides are oligonucleotides having nuclease resistance-conferring bulky substituents at their 3' and/or 5' end(s) and/or various other structural modifications not found *in vivo* without human intervention are also considered herein as modified.

In one preferred embodiment of the invention, the oligonucleotide administered includes at least one 2'-substituted ribonucleotide at its 3' terminus or 5' terminus.

For purposes of the invention, the term "2'-substituted oligonucleotide" refers to an oligonucleotide having a sugar attached to a chemical group other that a hydroxyl group at its 2' position. The 2'-OH of the ribose molecule can be substituted with -O-lower alkyl containing 1-6 carbon atoms, aryl or substituted aryl or allyl having 2-6 carbon atoms, e.g., 2'-O-allyl, 2'-O-aryl, 2'-O-alkyl (such as a 2'-O-methyl), 2'-halo, or 2'-amino, but not with 2'-H, wherein allyl, aryl, or alkyl groups may be unsubstituted or substituted, e.g., with halo, hydroxy, trifluoromethyl, cyano, nitro, acyl, acyloxy, alkoxy, carboxyl, carbalkoxyl or amino groups.

In some embodiments, all but four or five nucleotides at the 5' or 3' terminus of the oligonucleotide are 2'-substituted ribonucleotides. In other embodiments, the oligonucleotide has at least one 2'-substituted ribonucleotide at both its 3' and 5' termini, and in yet other embodiments, the oligonucleotide is composed of 2'-substituted ribonucleotides in all positions with the exception of at least four or five contiguous deoxyribonucleotide nucleotides in any interior position. Another aspect of the invention includes the administration of an oligonucleotide composed of nucleotides that are all 2'-substituted ribonucleotides. Particular embodiments include oligonucleotides having a 2'-O-alkyl-ribonucleotide such as a 2'-0 methyl.

In other embodiments, the oligonucleotide useful in the invention has at least one methylphosphonate deoxynucleotide at its 3' and 5' termini. In some preferred embodiments, the oligonucleotide has at least two methylphosphonate deoxynucleotides at the 3' terminus and at the 5' terminus. In particular embodiments, this oligonucleotide further comprises phosphorothioate internucleotide linkages.

In another embodiment of the invention, the oligonucleotide administered has at least one deoxyribonucleotide, and in a preferred embodiment, the oligonucleotide has at least four or five contiguous deoxyribonucleotides capable of activating RNase H.

The oligonucleotide administered is complementary to a gene of a virus, pathogenic organism, or a cellular gene in some embodiments of the invention. In some embodiments, the oligonucleotide is complementary to a gene of a virus involved in AIDS, oral or genital herpes, papilloma warts, influenza, foot and mouth disease, yellow fever, chicken pox, shingles, adult T-cell leukemia, Burkitt's lymphoma, nasopharyngeal carcinoma, or hepatitis. In one particular embodiment, the oligonucleotide is complementary to an HIV gene and includes about 15 to 26 nucleotides linked by phosphorothioate internucleotide linkages, at least one of the nucleotides at the 3' terminus being a 2'-substituted ribonucleotide, and at least four contiguous deoxyribonucleotides.

In another embodiment, the oligonucleotide is complementary to a gene encoding a protein in associated with Alzheimer's disease.

In yet other embodiments, the oligonucleotide is complementary to a gene encoding a protein expressed in a parasite that causes a parasitic disease such as amebiasis, Chagas' disease, toxoplasmosis, pneumocytosis, giardiasis, cryptoporidiosis, trichomoniasis, malaria, ascariasis, filariasis, trichinosis, or schistosomiasis infections.

In another aspect, the invention provides introducing an intact oligonucleotide into an animal. In this method an end-protected oligonucleotide is colorectally administered to the animal, whereby the oligonucleotide is present in intact form in the systemic plasma of the mammal at least about four hours following administration.

As used herein, the term "intact form" refers to an administered oligonucleotide which is relatively undegraded or undigested. This oligonucleotide is about 5 to 50, preferably about 12 to 35, and most preferably about 15 to 25 nucleotides in length.

An "end-protected oligonucleotide" is used herein to describe an oligonucleotide which has been modified at its 5' and/or 3' terminus such that it is less susceptible to enzymatic digestion by exonucleases than oligonucleotides which are not end-protected. Any modification to the terminus or termini of an administered oligonucleotide which results in protection from exonucleases but which does not greatly inhibit the ability of an oligonucleotide to hybridize to a complementary nucleotide sequence are meant to be encompassed by this term. In one embodiment, an end-protected oligonucleotide comprises at least one 2'-O-methyl-ribonucleotide or methylphosphonate deoxynucleotide at each terminus. In another embodiment the end-protected oligonucleotide comprises at least two 2'-O-methyl-ribonucleotides or methylphosphonate deoxynucleotides at each terminus. In yet another embodiment, the end-protected oligonucleotide comprises at least two 2'-O-methyl ribonucleotides at each terminus and further comprises phosphorothioate internucleoside linkages. In still another embodiment, the end-protected oligonucleotide comprises at least two methylphosphonate deoxynucleotides at each terminus and further comprises phosphorothioate internucleotide linkages. In some embodiments, the end-protected oligonucleotide comprises four methylphosphonate deoxynucleotides at each terminus.

### BRIEF DESCRIPTION OF THE DRAWINGS

The foregoing and other objects of the present invention, the various features thereof, as well as the invention itself may be more fully understood from the following description, when read together with the accompanying drawings in which:
FIG. 1 is a diagrammatic representation of the enterohepatic circulation of oligonucleotides and the delivery of such oligonucleotides through the gastrointestinal tract;
FIG. 2 is a schematic representation of the chemical structure of PS-oligonucleotide and end-modified MBO 1 (SEQ ID NO:11) and MBO 2 (SEQ ID NO:16);
FIG. 3A is an HPLC profile of radiolabelled MBO 1 standard;
FIG. 3B is an HPLC profile of radioactivity in the contents of the large intestine of a rat 4 hours after administration of radiolabelled MBO 1 to the large intestine of the rat;
FIG. 3C is an HPLC profile of radioactivity in the large intestine of a rat 4 hours after administration of radiolabelled MBO 1 to the large intestine of the rat;
FIG. 4 is a graphic representation of the concentration of MBO 1 equivalents in plasma at various times after administration of various dosages of MBO 1 to the large intestine of a rat;
FIG. 5 is a graphic representation of the concentration of MBO 1 equivalents in selected tissues 4 hours after administration of various dosages of MBO 1 to the large intestine of a rat;
FIG. 6A is an HPLC profile of radioactivity in the plasma of a rat 4 hours after administration of radiolabelled MBO 1 to the large intestine of the rat;
FIG. 6B is an HPLC profile of radioactivity in the liver of a rat 4 hours after administration of radiolabelled MBO 1 to the large intestine of the rat; and
FIG. 6C is an HPLC profile of radioactivity in the kidney of a rat 4 hours after administration of radiolabelled MBO 1 to the large intestine of the rat.

### DETAILED DESCRIPTION OF THE PREFERRED EMBODIMENTS

The patent and scientific literature referred to herein establishes the knowledge that is available to those with skill in the art.

The present invention provides a pharmaceutical formulation comprising an oligonucleotide of about 12 to about 50 nucleotides having non-phosphodiester internucleotide linkages and being complementary to a targeted gene, for colorectal administration to a mammal, wherein the oligonucleotide has 3' and 5' termini and further comprises at least one 2'-substituted ribonucleotide at the 5' terminus, at the 3' terminus, or at the 5' terminus and the 3' terminus.

In a further aspect the present invention provides the use of an oligonucleotide of about 15 to 25 nucleotides and comprising non-phosphodiester internucleotide linkages for the manufacture of a pharmaceutical formulation for colorectal administration for the treatment of virus infections, infections by pathogenic organisms, or diseases or disorders resulting from abnormal gene expression or from the expression of an abnormal gene product wherein the oligonucleotide has 3' and 5' termini and further comprises at least one 2'-substituted ribonucleotide at the 5' terminus, at the 3' terminus, or at the 5' terminus and the 3' terminus.

It is known that a synthetic oligonucleotide, called an "antisense oligonucleotide," can bind to a target single-stranded nucleic acid molecule according to the Watson-Crick or the Hoogsteen rule of base pairing, and in doing so, disrupt the function of the target by one of several mechanisms: by preventing the binding of factors required for normal transcription, splicing, or translation; by triggering the enzymatic destruction of mRNA by RNase H if a contiguous region of deoxyribonucleotides exists in the oligonucleotide, and/or by destroying the target via reactive groups attached directly to the antisense oligonucleotide.

Thus, because of the properties described above, such oligonucleotides are useful therapeutically by their ability to control or down-regulate the expression of a particular gene in an animal, according to the method of the present invention.

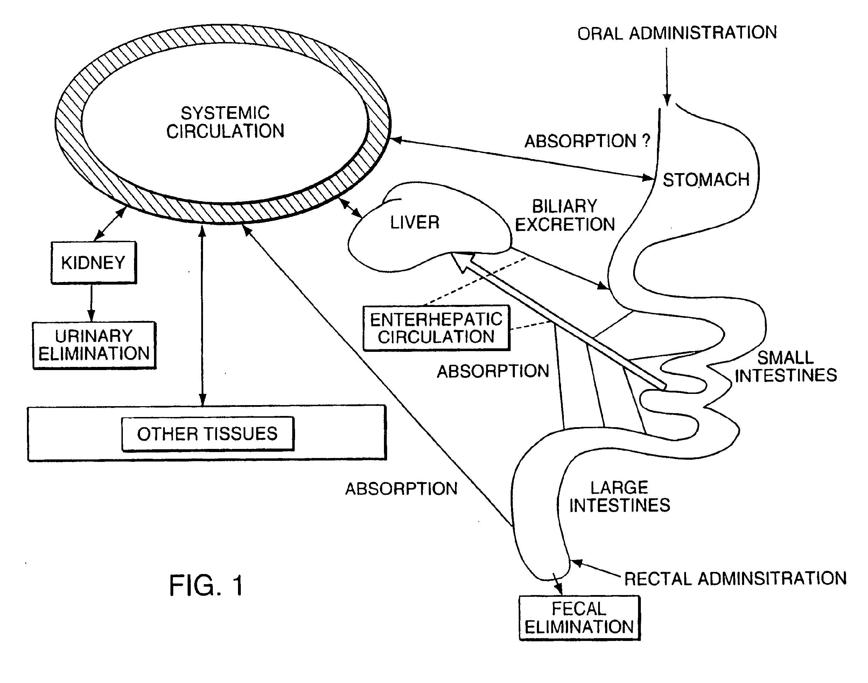

The pharmacokinetics and factors affecting gastrointestinal absorption of oligonucleotides, including colorectal absorption, are summarized in the scheme presented in FIG. 1. Briefly, when oligonucleotides are administered orally, they may be stable in the stomach contents and whether they are absorbed through the stomach wall is not clear. When the administered oligonucleotides move into small intestines, extensive degradation of PS-oligonucleotides and some degradation of MBO's may occur. Intact oligonucleotides (and maybe degradative forms) are absorbed through portal venous blood and enter the liver. The absorbed oligonucleotides may undergo metabolism in the liver (the first-pass effect) and enter the systemic circulation. Oligonucleotides and their metabolites are excreted into bile and enter the intestinal lumen and reenter the enterohepatic circulation. Oligonucleotides in the systemic circulation are distributed into various tissues and excreted into urine as seen following i.v. administration. When orally administered oligonucleotides move into the large intestine, most PS-oligonucleotides and some MBO'S may be present as degradation products. In general, oligonucleotides absorbed through the upper portion of the large intestine enter the liver, and oligonucleotides absorbed through the lower portion of the large intestine directly enter the systemic circulation. The latter are not metabolized in the liver and the first-pass effect of the liver is avoided. Colorectal administration of oligonucleotides takes the advantage of this opportunity. When oligonucleotides are administered into the rectum, most absorbed oligonucleotides enter the systemic circulation. Colorectal administration employs the pharmacokinetics of phosphorothioates as well as MBO's, making PS a viable choice for gastrointestinal administration.

In general, the following factors are important to the development of rectal oligonucleotides therapeutics: 1) stability of oligonucleotides in the gastrointestinal tract; 2) duration of the retention of oligonucleotides in the gastrointestinal tract; 3) the structure and physical and biochemical properties of oligonucleotides, e.g., charges; 4) the first-pass effect of the liver; 5) diet and host status of the gastrointestinal and hepatic functions; and 6) formulations. The advantages of delivery of oligonucleotides through rectal administration are obvious. The slow but continuous release of oligonucleotides into the systemic circulation may increase the uptake of target tissues. In addition, it avoids the high plasma concentrations associated with *i.v.* injection and reduces the risk of side effects resulting from these high concentrations.

The oligonucleotides which are colorectally administered according to the method of invention are at least 6 nucleotides in length, but are preferably 6 to 50 nucleotides long, with 15 to 30mers being the most common. They are composed of deoxyribonucleotides, ribonucleotides, or a combination of both, with the 5' end of one nucleotide and the 3' end of another nucleotide being covalently linked by phosphodiester bonds.

The oligonucleotides used in the present invention may also be modified in a number of ways without compromising their ability to hybridize to the target nucleic acid. Such modifications include, for example, non-phosphodiester internucleotide linkages including alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters. Particularly useful oligonucleotides are linked with phosphorothioate and/or phosphorodithioate internucleoside linkages. Preferably, oligonucleotides according to the invention ranging from about 6 to about 50 nucleotides in length, and most preferably from about 12 to about 30 nucleotides in length, will have from 11 to 29 non-phosphodiester internucleotide linkages.

Other useful modifications include those which are internal or at the end(s) of the oligonucleotide molecule and include additions to the molecule of the internucleoside phosphate linkages, such as cholesteryl or diamine compounds with varying numbers of carbon residues between the amino groups and terminal ribose, deoxyribose and phosphate modifications which cleave, or crosslink to the opposite chains or to associated enzymes or other proteins which bind to the viral genome. Examples of such modified oligonucleotides include oligonucleotides with a modified base and/or sugar such as arabinose instead of ribose, or a 3', 5'-substituted oligonucleotide having a sugar which, at both its 3' and 5' positions is attached to a chemical group other than a hydroxyl group (at its 3' position) and other than a phosphate group (at its 5' position). Other modified oligonucleotides are capped with a nuclease resistance-conferring bulky substituent at their 3' and/or 5' end(s), or have a substitution in one nonbridging oxygen per nucleotide. Such modifications can be at some or all of the internucleoside linkages, as well as at either or both ends of the oligonucleotide and/or in the interior of the molecule. Oligonucleotides which are self-stabilized are also considered to be modified oligonucleotides useful in the methods of the invention (Tang et al. (1993) *Nucleic Acids Res.* 20:2729-2735). These oligonucleotides comprise two regions: a target hybridizing region; and a self-complementary region having an oligonucleotide sequence complementary to a nucleic acid sequence that is within the self-stabilized oligonucleotide.

Modified and unmodified oligonucleotides can be prepared according to known methods which can be carried out manually or by an automated synthesizer as described by Brown *(A Brief History of Oligonucleotide Synthesis. Protocols for Oligonucleotides and Analogs, Methods in Molecular Biology* (1994) 20:1-8). See also, Sonveaux "Protecting Groups in Oligonucleotides Synthesis" in Agrawal (1994) *Methods in Molecular Biology* 26:1-72; Agrawal et al. (1992) *Trends Biotechnol.* 10:152-158; Uhlmann et al. (1990) *Chem. Rev.* 90:543-583; and (1987) *Tetrahedron. Lett.* 28:(31):3539-3542).

One preferred oligonucleotide useful in the method of the invention are hybrid oligonucleotides containing both deoxyribonucleotides and at least one 2' substituted ribonucleotide. For purposes of the invention, the term "2'-substituted" means substitution of the 2'-OH of the ribose molecule with, e.g., 2'-O-allyl, 2'-O-alkyl, 2'-halo, or 2'-amino, but not with 2'-H, wherein allyl, aryl, or alkyl groups may be unsubstituted or substituted, e.g., with halo, hydroxy, trifluoromethyl, cyano, nitro, acyl, acyloxy, alkoxy, carboxyl, carbalkoxyl or amino groups. Other preferred oligonucleotides useful in the method of the invention have at least one or all phosphorothioate internucleotide linkages.

The hybrid DNA/RNA oligonucleotides useful in the method of the invention resist nucleolytic degradation, form stable duplexes with RNA or DNA, and preferably activate RNase H when hybridized with RNA. They may additionally include at least one unsubstituted ribonucleotide. For example, an oligonucleotide useful in the method of the invention may contain all deoxyribonucleotides with the exception of one 2' substituted ribonucleotide at the 3' terminus of the oligonucleotide. Alternatively, the oligonucleotide may have at least one substituted ribonucleotide at both its 3' and 5' termini.

One preferred class of oligonucleotides useful in the method of the invention contains at least four or more deoxyribonucleotides in a contiguous block, so as to provide an activating segment for RNase H. In certain cases, more than one such activating segment will be present at any location within the oligonucleotide. There may be a majority of deoxyribonucleotides in oligonucleotides useful in the method of the invention. In fact, such oligonucleotides may have as many as all but one, two, three, or four nucleotide(s) being deoxyribonucleotides. Thus, in a preferred oligonucleotide having from about 6 to about 50 nucleotides or most preferably from about 12 to about 30 nucleotides, the number of deoxyribonucleotides present ranges from 1 to about 29.

Other useful oligonucleotides may consist particularly of at least one, two, four, or more 2'-substituted ribonucleotide(s) at one or both termini of the oligonucleotide. Some oligonucleotides useful in the method of the invention have only 2'-substituted ribonucleotides. The 2' substituted ribonucleotide(s) in the oligonucleotide may contain at the 2' position of the ribose, a -O-lower alkyl containing 1-6 carbon atoms, aryl or substituted aryl or allyl having 2-6 carbon atoms e.g., 2'-O-allyl, 2'-O-aryl, 2'-O-alkyl, 2'-halo, or 2'-amino, but not with 2'-H, wherein allyl, aryl, or alkyl groups may be unsubstituted or substituted, e.g., with halo, hydroxy, trifluoromethyl, cyano, nitro, acyl, acyloxy, alkoxy, carboxyl, carbalkoxyl or amino groups. Useful substituted ribonucleotides are 2'-0-alkyls such as 2'-0-methyl.

TABLE 1 lists some representative species of oligonucleotides which are useful in the method of the invention. 2'-substituted nucleotides are underscored, and nucleotide methylphosphonates are bolded.

**TABLE 1**

| NO. | OLIGONUCLEOTIDE | SEQ ID NO: |
|---|---|---|
| 1 | CTCTCGCACCCATCTCTCTCCTTCU | 1 |
| 2 | CTCTCGCACCCATCTCTCTCCTUCU | 2 |
| 3 | CTCTCGCACCCATCTCTCTCCUUCU | 3 |
| 4 | CTCTCGCACCCATCTCUCUCCUUCU | 4 |
| 5 | CTCTCGCACCCAUCUCUCUCCUUCU | 5 |
| 6 | CTCTCGCACCCAUCUCUCUCCUUCU | 5 |
| 7 | CTCTCGCACCCAUCUCUCUCCUUCU | 5 |
| 8 | CUCUCGCACCCAUCUCUCUCCUUCU | 6 |
| 9 | CTCTCGCACCCATCTCTCTCCTTCU | 7 |
| 10 | CUCTCGCACCCATCTCTCTCCTTCU | 7 |
| 11 | CUCUCGCACCCATCTCTCTCCUUCU | 8 |
| 12 | CUCUCGCACCCATCTCUCUCCUUCU | 9 |
| 13 | CUCUCGCACCCAUCUCUCUCCUUCU | 10 |
| 14 | CUCUCGCACCCATCTCTCUCCUUCU | 11 |
| 15 | CTCTCGCACCCAUCUCUCUCCUUCU | 5 |
| 16 | CUCUCGCACCCAUCTCTCTCCUUCU | 12 |
| 17 | CUCUCGCACCCATCTCTCTCCUUCU | 13 |
| 18 | CUCTCGCACCCAUCUCUCUCCUUCU | 14 |
| 19 | CUCTCGCACCCATCTCTCUCCUUCU | 15 |
| MBO 1 | CUCUCGCACCCATCTCTCTCCUUCU | 11 |
| MBO 2 | CTCTCGCACCCATCTCTCTCCTTCT | 16 |
| 20 | CTCTCGCACCCATCTCTCTCCTTCT | 17 |
| 21 | CUCTCGCACCCATCTCTCTCCTTCT | 18 |
| 22 | CUCUCGCACCCATCTCTCTCCTTCT | 19 |
| 23 | CUCUCGCACCCATCTCTCTCCTTCT | 19 |
| 24 | CUCUCGCACCCAUCUCTCTCCTTCT | 20 |
| 25 | CUCUCGCACCCAUCUCUCUCCTTCT | 21 |
| 26 | CTCTCGCACCCATCTCTCTCCTTCT | 17 |

The oligonucleotides used in the invention are effective in inhibiting the expression of various genes in viruses, pathogenic organisms, or in inhibiting the expression of cellular genes. The ability to inhibit such agents is clearly important to the treatment of a variety of disease states. Thus, oligonucleotides according to the method of the invention have a nucleotide sequence which is complementary to a nucleic acid sequence that is from a virus, a pathogenic organism or a cellular gene.

For purposes of the invention, the term "oligonucleotide sequence that is complementary to a nucleic acid sequence" is intended to mean an oligonucleotide sequence that binds to the target nucleic acid sequence under physiological conditions, e.g., by Watson-Crick base pairing (interaction between oligonucleotide and single-stranded nucleic acid, such as RNA or cDNA) or by Hoogsteen base pairing (interaction between oligonucleotide and double-stranded nucleic acid) or by any other means including in the case of a oligonucleotide binding to RNA, pseudoknot formation. Such binding (by Watson Crick base pairing) under physiological conditions is measured as a practical matter by observing interference with the function of the nucleic acid sequence. The nucleic acid to which the oligonucleotide is complementary may be genomic DNA, RNA, mRNA or cDNA.

The sequence of the nucleic acid to which an oligonucleotide according to the invention is complementary will vary, depending upon the gene to be down-regulated. In some cases, the target gene or nucleic acid sequence will be a virus nucleic acid sequence. The use of antisense oligonucleotides to inhibit various viruses is well known (reviewed in Agrawal (1992) *Trends in Biotech.* 10:152-158). Viral nucleic acid sequences that are complementary to effective antisense oligonucleotides have been described for many viruses, including human immunodeficiency virus type 1 (HIV-1) (U.S. Patent No. 4,806,463), herpes simplex virus (U.S. Patent No. 4,689,320), influenza virus (U.S. Patent No. 5,194,428), and human papilloma virus (Storey et al. (1991) *Nucleic Acids Res*. 19:4109-4114). Sequences complementary to any of these nucleic acid sequences can be used for oligonucleotides according to the invention, as can be oligonucleotide sequences complementary to nucleic acid sequences from any other virus. Additional viruses that have known nucleic acid sequences against which antisense oligonucleotides can be prepared include, but are not limited to, foot and mouth disease virus (see, Robertson et al. (1985) *J. Virol*. 54:651; Harris et al. *(1980) Virol.* 36:659), yellow fever virus (see Rice et al. (1985) *Science* 229:726), varicella-zoster virus (see, Davison and Scott (1986) *J. Gen. Virol.* 67:2279), Epstein-Barr virus, cytomegalovirus, respiratory syncytial virus (RSV), and cucumber mosaic virus (see Richards et al. (1978) *Virol.* 89:395).

For example, an oligonucleotide has been designed which is complementary to a portion of the HIV-1 gene, and as such, has significant anti-HIV effects (Agrawal (1992) *Antisense Res. Development* 2:261-266). The target of this oligonucleotide has been found to be conserved among various HIV-1 isolates. It is 56% G + C rich, water soluble, and relatively stable under physiological conditions. This oligonucleotide binds to a complementary RNA target under physiological conditions, with the T of the duplex approximately being 56°C. The antiviral activity of this oligonucleotide has been tested in several models, including acutely and chronically infected CEM cells, long-term cultures mimicking *in vivo* conditions, human peripheral blood lymphocytes and macrophages, and isolates from HIV-1 infected patients (Lisziewicz et al. (*Proc. Natl. Acad. Sci. (USA)* (1992) 89:11209-11213); Lisziewicz et al. (*Proc. Natl. Acad. Sci. (USA)* (1993) 90:3860-3864); Lisziewicz et al. (*Proc. Natl. Acad. Sci. (USA)* (1994) 91:7942-7946); Agrawal et al. (*J. Ther. Biotech*) in press) .

The oligonucleotides according to the invention alternatively can have an oligonucleotide sequence complementary to a nucleic acid sequence of a pathogenic organism. The nucleic acid sequences of many pathogenic organisms have been described, including the malaria organism, *Plasmodium falciparum*, and many pathogenic bacteria. Oligonucleotide sequences complementary to nucleic acid sequences from any such pathogenic organism can be used in oligonucleotides according to the invention. Examples of pathogenic eucaryotes having known nucleic acid sequences against which antisense oligonucleotides can be prepared include *Trypanosom abrucei gambiense* and *Leishmania* (See Campbell et al., *Nature* 311:350 (1984)), *Fasciola hepatica* (See Zurita et al., *Proc. Natl. Acad. Sci. USA* 84:2340 (1987).

Antifungal oligonucleotides can be prepared using a target hybridizing region having an oligonucleotide sequence that is complementary to a nucleic acid sequence from, e.g., the chitin synthetase gene, and antibacterial oligonucleotides can be prepared using, e.g., the alanine racemase gene. Among fungal diseases that may be treatable by the method of treatment according to the invention are candidiasis, histoplasmosis, cryptococcocis, blastomycosis, aspergillosis, sporotrichosis, chromomycosis, dermatophytosis, and coccidioidomycosis. The method might also be used to treat rickettsial diseases (e.g., typhus, Rocky Mountain spotted fever), as well as sexually transmitted diseases caused by Chlamydia trachomatis or Lymphogranuloma venereum. A variety of parasitic diseases may be treated by the method according to the invention, including amebiasis, Chagas' disease, toxoplasmosis, pneumocystosis, giardiasis, cryptosporidiosis, trichomoniasis, and Pneumocystis carini pneumonia; also worm (helminthic) diseases such as ascariasis, filariasis, trichinosis, schistosomiasis and nematode or cestode infections. Malaria may be treated by the method of treatment of the invention regardless of whether it is caused by P. falcip arum, P. vivas, P. orale, or P. malariae.

The infectious diseases identified above may all be treated by the method of treatment according to the invention because the infectious agents for these diseases are known and thus oligonucleotides according to the invention can be prepared, having oligonucleotide sequence that is complementary to a nucleic acid sequence that is an essential nucleic acid sequence for the propagation of the infectious agent, such as an essential gene.

Other disease states or conditions that may be treatable by the method according to the invention are those which result from an abnormal expression or product of a cellular gene. These conditions may be treated by administration of oligonucleotides according to the invention, and have been discussed earlier in this disclosure.

Other oligonucleotides according to the invention can have a nucleotide sequence complementary to a cellular gene or gene transcript, the abnormal expression or product of which results in a disease state. The nucleic acid sequences of several such cellular genes have been described, including prion protein (Stahl et al. (1991) *FASEB J*. 5:2799-2807), the amyloid-like protein associated with Alzheimer's disease (U.S. Patent No. 5,015,570), and various well-known oncogenes and proto-oncogenes, such as c-*myb*, c-*myc*, c-*abl*, and n-*ras.* In addition, oligonucleotides that inhibit the synthesis of structural proteins or enzymes involved largely or exclusively in spermatogenesis, sperm motility, the binding of the sperm to the egg or any other step affecting sperm viability may be used as contraceptives. Similarly, contraceptives for women may be oligonucleotides that inhibit proteins or enzymes involved in ovulation, fertilization, implantation or in the biosynthesis of hormones involved in those processes.

Hypertension may be controlled by oligonucleotides that down-regulate the synthesis of angiotensin converting enzyme or related enzymes in the renin/angiotensin system. Platelet aggregation may be controlled by suppression of the synthesis of enzymes necessary for the synthesis of thromboxane A2 for use in myocardial and cerebral circulatory disorders, infarcts, arteriosclerosis, embolism and thrombosis. Deposition of cholesterol in arterial wall may be inhibited by suppression of the synthesis of fatty acid co-enzyme A: cholesterol acyl transferase in arteriosclerosis. Inhibition of the synthesis of cholinephosphotransferase may be useful in hypolipidemia.

There are numerous neural disorders in which hybridization arrest may be used to reduce or eliminate adverse effects of the disorder. For example, suppression of the synthesis of monoamine oxidase may be used in Parkinson's disease. Suppression of catechol o-methyl transferase may be used to treat depression; and suppression of indole N-methyl transferase may be used in treating schizophrenia.

Suppression of selected enzymes in the arachidonic acid cascade which leads to prostaglandins and leukotrienes may be useful in the control of platelet aggregation, allergy, inflammation, pain and asthma.

Suppression of the protein expressed by the multidrug resistance (*mdr*-1) gene, which can be responsible for development of resistance of tumors to a variety of anti-cancer drugs and is a major impediment in chemotherapy may prove to be beneficial in the treatment of cancer. Oligonucleotide sequences complementary to nucleic acid sequences from any of these genes can be used for oligonucleotides according to the invention, as can be oligonucleotide sequences complementary to any other cellular gene transcript, the abnormal expression or product of which results in a disease state.

The oligonucleotides described herein are administered colorectally to the animal subject in the form of therapeutic pharmaceutical formulations that are effective for treating virus infection, infections by pathogenic organisms, or disease or disorder resulting from abnormal gene expression or from the expression of an abnormal gene product and are suitable for colorectal delivery. In some aspects of the method according to the invention, the oligonucleotides are administered in conjunction with other therapeutic agents, e.g., AZT in the case of AIDS.

The therapeutic pharmaceutical formulation of the invention includes an oligonucleotide as described above and a physiologically acceptable carrier, such as an inert diluent or an assimilable carrier with which the oligonucleotide is administered. Suitable formulations that include pharmaceutically acceptable excipients for introducing compounds to the bloodstream by other than injection routes can be found in *Remington's Pharmaceutical Sciences* (18th ed.) (Genarro, ed. (1990) Mack Publishing Co., Easton, PA). The pharmaceutical formulation that may be introduced in a solid, semi-solid, suspension, or emulsion form and may be compounded with any number of well-known, pharmaceutically acceptable additives. The oligonucleotide and other ingredients may be enclosed in a hard or soft shell gelatin capsule, contained within gels or creams, or compressed into suppositories, and the like. Sustained release delivery systems and/or coatings for colorectally administered dosage forms are also contemplated, such as those described in U.S. Patent Nos. 4,704,295, 4,556,552, 4,309,404, and 4,309,406 for oral administration.

As used herein, the term "therapeutically effective amount" means the total amount of each active component of the pharmaceutical formulation or method that is sufficient to show a meaningful subject or patient benefit, i.e., healing of disease conditions characterized by the disease being treated and/or an increase in rate of healing of such conditions, a reduction in the expression of proteins or cells which cause or characterize the disease or disorder being treated (e.g., in the case of a virus, a decrease in virus load over baseline under disease conditions. When applied to an individual active ingredient, administered alone, the term refers to that ingredient alone. When applied to a combination, the term refers to combined amounts of the active ingredients that result in the therapeutic effect, whether administered in combination, serially or simultaneously.

The therapeutically effective amount of synthetic oligonucleotide colorectally administered in the method of the invention will depend upon the nature and severity of the condition being treated, and on the nature of prior treatments which the patent has undergone. Ultimately, the attending physician will decide the amount of synthetic oligonucleotide with which to treat each individual patient. Initially, the attending physician may administer low doses of the synthetic oligonucleotide and observe the patient's response. Larger doses of synthetic oligonucleotide may be administered until the optimal therapeutic effect is obtained for the patient, and at that point the dosage is not increased further. It is contemplated that the dosages of the pharmaceutical compositions administered in the method of the present invention should contain about 0.1 to 100.0 mg/kg body weight per day, preferably 0.1 to 75.0 mg/kg body weight per day, more preferably, 1.0 to 50.0 mg/kg body weight per day, even more preferably, 1 to 25 mg/kg body weight per day, and even more preferably, 1 to 10 or 1 to 5.0 mg/kg body weight per day. The oligonucleotide is preferably administered at a sufficient dosage to attain a blood level of oligonucleotide from about 0.01 µM to about 100 µM. Preferably, the concentration of oligonucleotide at the site of aberrant gene expression should be from about 0.01 µM to about 50 µM, more preferably, from about 0.01 µM to about 10 µM, and most preferably from about 0.05 µM to about 5 µM. However, for localized administration, much lower concentrations than this may be effective, and much higher concentrations may be tolerated. It may be desirable to administer simultaneously or sequentially a therapeutically effective amount of one or more of the therapeutic compositions of the invention when individual as a single treatment episode.

It will be appreciated that the unit content of active ingredient or ingredients contained in an individual dose of each dosage form need not in itself constitute an effective amount since the necessary effective amount can be reached by administration of a plurality of dosage units (such as suppositories, gels, or creams, or combinations thereof). In fact, multi-dosing (once a day) has been shown to significantly increase the plasma and tissue concentrations of MBO's (data not shown).

The oligonucleotides according to the invention are administered to the animal in a therapeutically effective manner. A "therapeutically effective manner" refers to a route, duration, and frequency of administration of the pharmaceutical formulation which ultimately results in meaningful patient benefit, as described above. In some embodiments of the invention, the pharmaceutical formulation is administered in bolus, continuous, intermittent, or continuous amounts, followed by intermittent regimens.

The pharmaceutical formulation can be administered in bolus, continuous, or intermittent dosages, or in a combination of continuous and intermittent dosages, as determined by the physician and the degree and/or stage of illness of the patient. The duration of therapy using the pharmaceutical composition of the present invention will vary, depending on the unique characteristics of the oligonucleotide and the particular therapeutic effect to be achieved, the limitations inherent in the art of preparing such a therapeutic formulation for the treatment of humans, the severity of the disease being treated and the condition and potential idiosyncratic response of each individual patient. Ultimately the attending physician will decide on the appropriate duration of intravenous therapy using the pharmaceutical composition of the present invention.

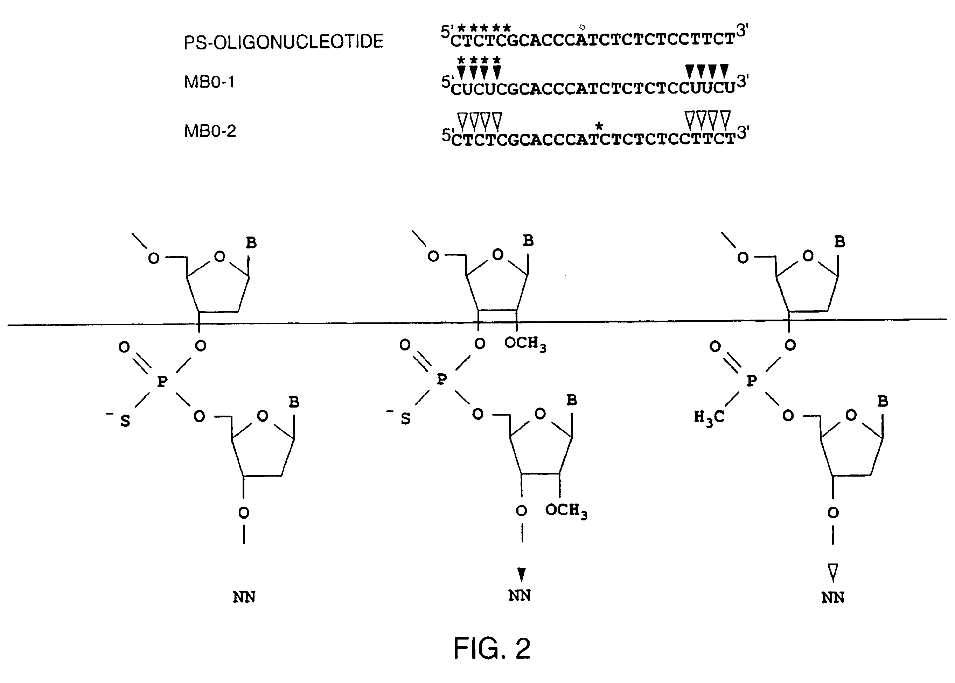

To determine the stability of antisense oligonucleotides according to the invention in the intestinal tract, and to determine their ability to be absorbed through the intestinal wall, two radioactively labelled, end-modified oligonucleotides, MBO 1 (SEQ ID NO:11) and MBO 2 (SEQ ID NO:16), and one phosphorothioate oligonucleotide (SEQ ID NO:16) were administered to the large intestine of rats. The chemical structure of these oligonucleotides is shown in FIG. 2. The tissue distribution of these oligonucleotides and their stability was then measured.

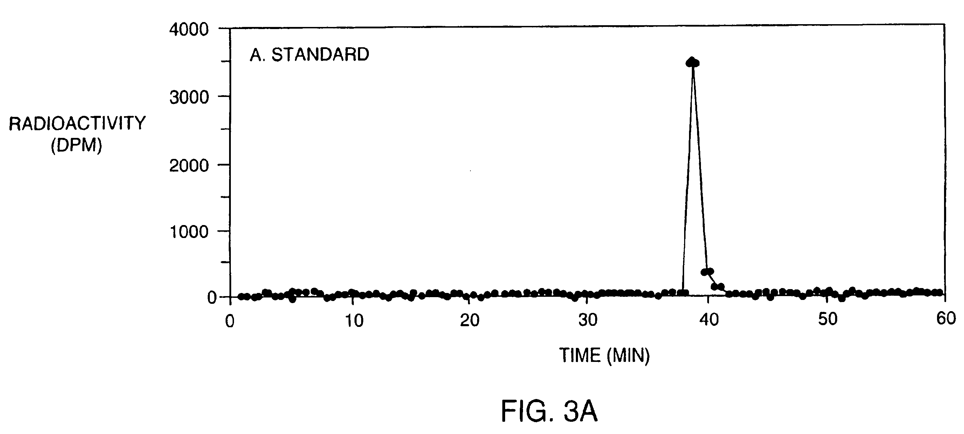

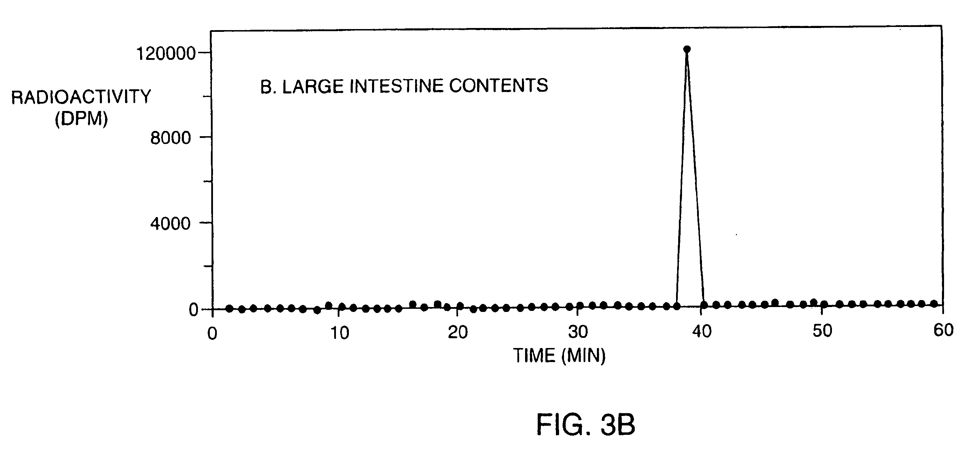

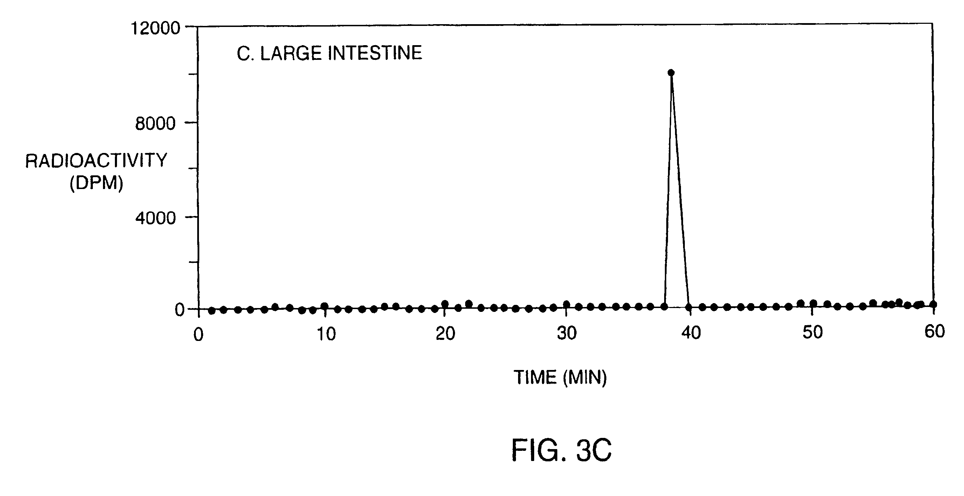

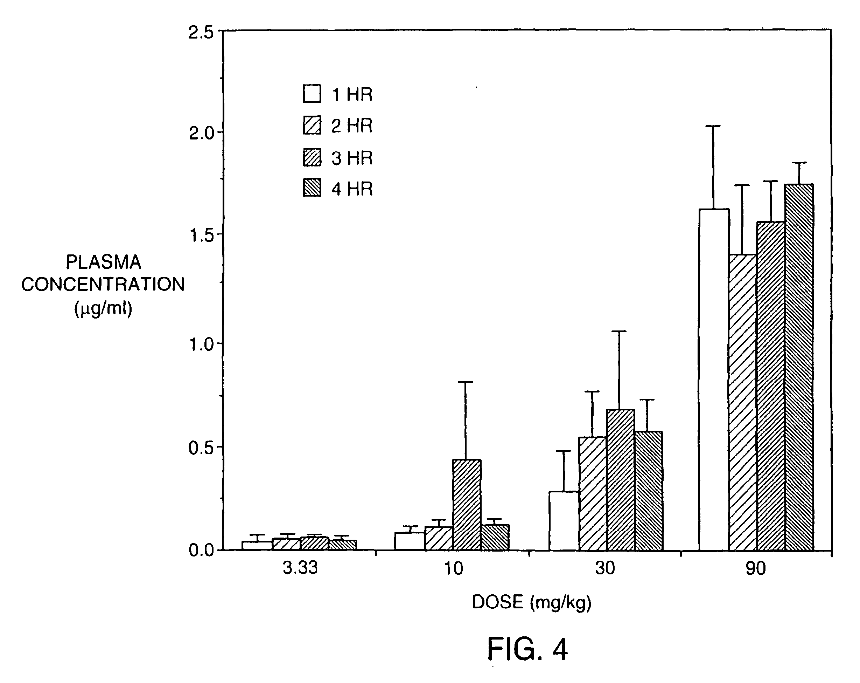

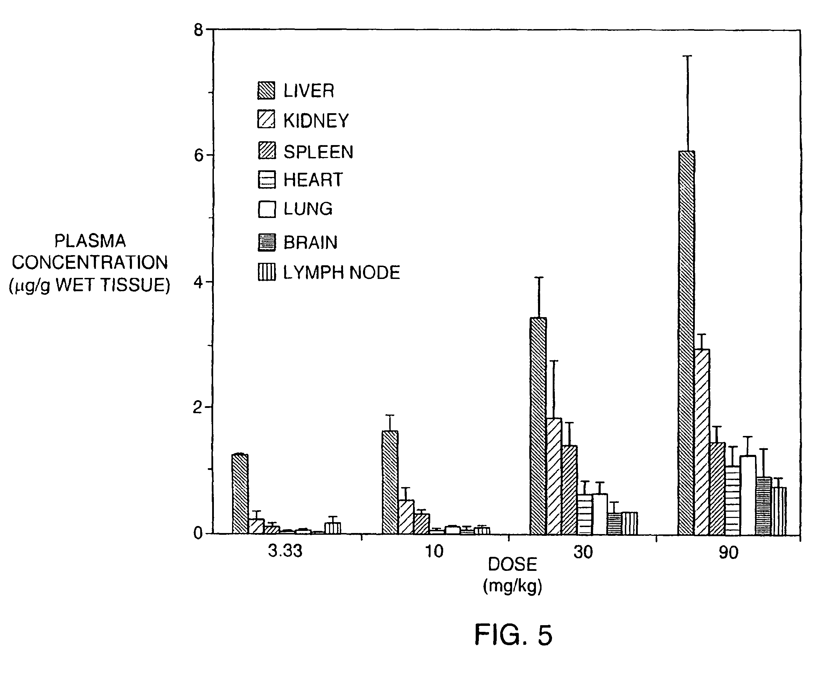

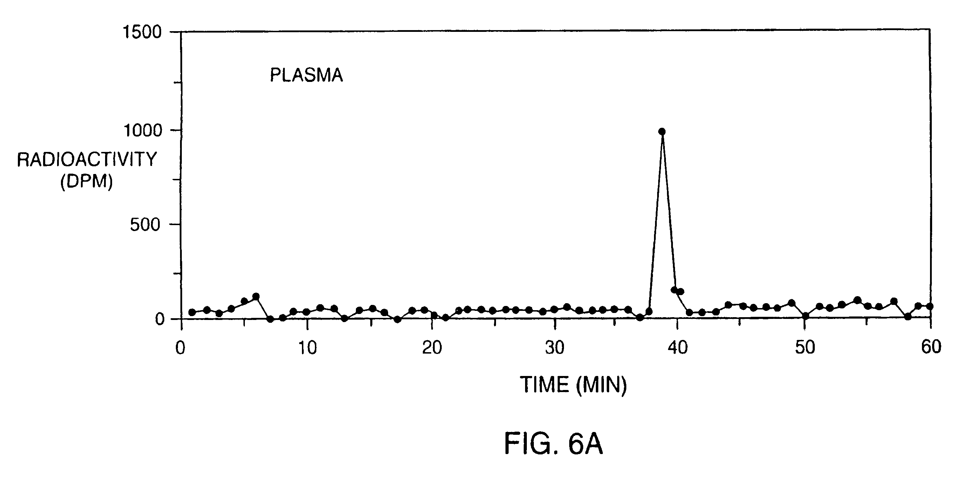

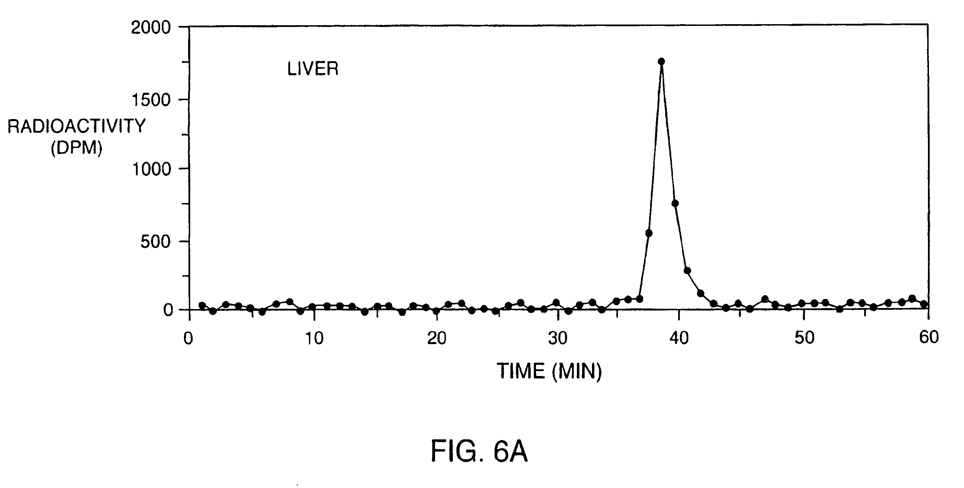

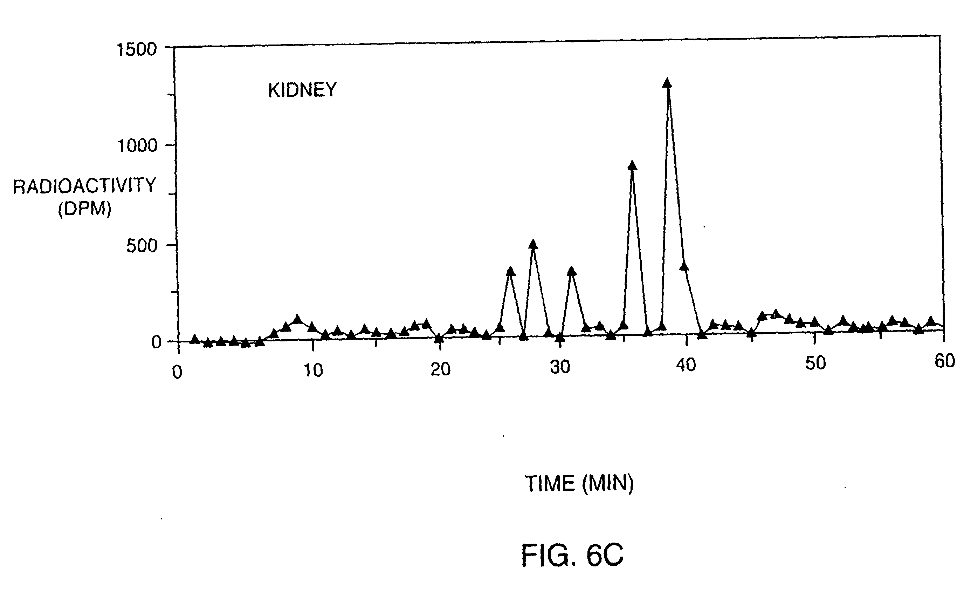

MBO 1 was stable in the large intestine as analyzed by HPLC and PAGE for up to 4 hr following administration, with minimal degradation being observed (FIGS. 3B and 4). Gel electrophoresis revealed that the majority of extracted radioactivity in large intestine and its contents was intact oligonucleotide (data not shown). The absorption of MBO 1 was examined at doses of 3.3, 10, 30, and 90 mg/kg. Oligonucleotide-derived radioactivity was detectable in various tissues following large intestinal administration of the radiolabeled MBO 1. FIG. 4 illustrates the concentration of the MBO 1 equivalents in plasma, indicating that the oligonucleotide was absorbed in a time- and concentration-dependent fashion. Significant accumulation of oligonucleotide- derived radioactivity was observed in various tissues. FIG. 5 illustrates the concentration of MBO 1 equivalents in selected tissues, including kidney, liver, spleen, bone marrow, lymph node, and brain, 4 hr after administration. As can be seen in FIGS. 6B and 6C, HPLC analysis revealed both intact and degraded forms of MBO 1 in kidney, but the majority of the radioactivity in the liver and kidneys was associated with the intact form of MBO 1. Gel electrophoresis also revealed the majority of the extracted radioactivity in these samples was associated with the intact form of MBO 1 (data not shown). No significant degraded products were detected in large intestine for up to 4 hr after administration. Approximately 4 to 14% of administered MBO 1 was absorbed within 4 hr in the anesthetized animals, depending on the dose levels.

In separate studies, similar results were obtained following rectal administration of PS-oligonucleotide and MBO 2. At 10 mg/kg, PS-oligonucleotide had a 4-hr absorption ratio of 8.74% of the administered dose, and MBO 2 had a ratio of 6.6% of the administered dose (data not shown).

Previous studies described in Zhang et al. (*Clin. Chem.* (1995) 41:863-873), demonstrated that, following oral administration, PS-oligonucleotides could be well absorbed through the gastrointestinal wall, but were extensively degraded in the liver; little intact PS-oligonucleotides were available, therefore, in the systemic tissues. Rectal delivery avoids the first-pass effect in the liver. Following large intestine administration, PS-oligonucleotide was well absorbed largely in the intact form and was less extensively degraded in other tissues. Furthermore, because absorption rates were estimated in anesthetized rats, the actual bioavailability of colorectal oligonucleotides may be underestimated.

These studies represent the first reports on the bioavailability of antisense oligonucleotides following colorectal administration in experimental animals. They show that, following large intestine administration: 1) PS-oligonucleotide and end-modified oligonucleotides were stable in the large intestinal lumen; 2) they were absorbed through the large intestine wall; 3) the absorbed oligonucleotide-derived radioactivity was widely distributed to various tissues with a pattern similar to that seen following i.v. administration; and 4) radioactivity in tissues such as liver and kidneys was associated with intact oligonucleotide as well as metabolites.

Thus, using the method of the invention, successful absorption of oligonucleotides was accomplished through the intestinal tract and distributed throughout the body. Intact oligonucleotides were detected in plasma and various tissues. These results demonstrate that colorectal administration is a potential means for delivery of oligonucleotides as therapeutic agents.

These results also demonstrate that synthetic oligonucleotides can be introduced in intact form into a mammal, and that such an oligonucleotides can be found in intact form at least four hours after colorectal administration in intact form in systemic plasma and in other organs and tissues.

The following examples illustrate the preferred modes of making and practicing the present invention, but are not meant to limit the scope of the invention since alternative methods may be utilized to obtain similar results.

### EXAMPLES

### 1. Synthesis and Analysis of Oligonucleotide

Hybrid 25-mer phosphorothioate-linked oligonucleotides having SEQ ID NO:1 and 11 and containing 2'-0-methyl ribonucleotide 3' and 5' sequences and a deoxyribonucleotide interior was synthesized, purified, and analyzed as follows.

Unmodified phosphorothioate deoxynucleosides were synthesized on CPG on a 5-6 µmole scale on an automated synthesizer (model 8700, Millipore, Bedford, MA) using the H-phosphonate approach described in U.S. Patent No. 5,149,798. Deoxynucleoside H-phosphonates were obtained from Millipore (Bedford, MA). 2'-O-methyl ribonucleotide H-phosphonates or phosphorothioates were synthesized by standard procedures (see, e.g., "Protocols for Oligonucleotides and Analogs" in *Meth. Mol. Biol*. (1993) volume 20) or commercially obtained (e.g., from Glenn Research, Sterling, VA and Clontech, Palo Alto, CA). Segments of oligonucleotides containing 2'-O-methyl nucleoside(s) were assembled by using 2'-O-methyl ribonucleoside H-phosphonates or phosphorothioates for the desired cycles. Similarly, segments of oligonucleotides containing deoxyribonucleosides were assembled by using deoxynucleoside H-phosphonates for the desired cycles. After assembly, CPG bound oligonucleotide H-phosphonate was oxidized with sulfur to generate the phosphorothioate linkage. Oligonucleotides were then deprotected in concentrated NH₄OH at 40°C for 48 hours.

Crude oligonucleotide (about 500 A₂₆₀ units) was analyzed on reverse low pressure chromatography on a C₁₈ reversed phase medium. The DMT group was removed by treatment with 80% aqueous acetic acid, then the oligonucleotides were dialyzed against distilled water and lyophilized.

A 2'-O-methyl end-protected oligonucleotide shown in FIG. 2 and having SEQ ID NO:16 was prepared as described in Agrawal and Tang *(Antisense Res. Dev.* (1992)2:261-66), Padmapriya et al. *(Antisense Res. Dev.* (1994) 4:185-199), Zhang et al. *(Biochem. Pharmacol*. (1995) 50:545-556; and Zhang et al. (J. *Pharm. Exp. Ther*. (1996) 278:971-979).

### 2. Radioactive Labelling of Oligonucleotide

To obtain ³⁵S-labelled oligonucleotide, synthesis was carried out in two steps. The first 19 nucleotides of the sequence SEQ ID NO:1) from its 3'-end were assembled using the β-cyanoethyl-phosphoramidite approach (see, Beaucage in *Protocols for Oligonucleotides and Analogs* (Agrawal, ed.), Humana Press, (1993), pp. 33-61). The last six nucleotides were assembled using the H-phosphonate approach (see, Froehler in *Protocols for Oligonucleotides and Analogs* (Agrawal, ed.) Humana Press, 1993, pp. 63-80). Controlled pore glass (CPG) support-bound oligonucleotide (30 mg of CPG; approximately 1 µM) containing five H-phosphonate linkage was oxidized with ³⁵S₈ (4 mCi, 1 Ci/mg, Amersham; 1 Ci = 37 GBq) in 60 ml carbon disulfide/pyridine/triethylamine (10:10:1). The oxidation reaction was performed at room temperature for 1 hr with occasional shaking. Then 2 µl, 5 µl, and 200 µl of 5% cold sulfur (³²S₈) in same solvent mixture was added every 30 min to complete the oxidation. The solution was removed and the CPG support was washed with carbon disulfide/pyridine/triethylamine (10:10:1) (3 x 500 µl) and with acetonitrile (3 x 700 µl). The product was deprotected in concentrated ammonium hydroxide (55°C, 14 hr) and evaporated. The resultant product was purified by polyacrylamide gel electrophoresis (20% polyacrylamide containing 7 M urea). The desired band was excised under UV shadowing and the PS-oligonucleotide was extracted from the gel and desalted with a Sep-Pak C18 cartridge (Waters) and Sephadex G-15 column. The yield was 20 A₂₆₀ units (600 µg; specific activity, 1 µCi/µg).

### 3. Animals and Drug Treatment

Male Sprague-Dawley rats (150-200 g, Harlan Laboratories, Indianapolis, IN) were used in the study. The animals were fed with commercial diet and water *ad libitum* for 1 week prior to the study. After each animal was anesthetized using pentobarbital, an incision was made on the lower part of the abdomen to expose the large intestine. The colon was cut open at the position of 0.5 cm to caecum. The large intestine contents were washed out using 30 ml of physiological saline (0.9% NaCl) at 37°C. After the anus was ligated, unlabelled and [³⁵S]-labelled oligonucleotides dissolved in physiological saline (0.9% NaCl) at designated concentrations were injected into the large intestine through the cut that was ligated after drug administration. The abdomen was then closed and the body temperature was maintained at 38 ± 0.5°C by means of a heat lamp.

Oligonucleotides were administered to rats at four dose levels, i.e., 3.3, 10, 30, and 90 mg/kg (3 rats per dose level). Blood samples were collected in heparinized tubes from animals at the various times, i.e., 1, 2, 3, and 4 hrs. Plasma was separated by centrifugation. At 4 hr after drug administration, animals were euthanized by exsanguination under sodium pentobarbital anesthesia. Following euthanasia, all tissue/organs were collected, immediately blotted on Whatman No. 1 filter paper, trimmed of extraneous fat or connective tissue, emptied and cleaned of all contents, and individually weighed prior to quantitation of oligonucleotide-derived radioactivity. Biological samples were analyzed by determination of total radioactivity, HPLC, and PAGE analysis using the methods described above.

### 4. Total Radioactivity Measurements

The total radioactivities in tissues and body fluids were determined by liquid scintillation spectrometry (LS 6000TA, Beckman, Irvine, CA). In brief, biological fluids (plasma, 50-100 µl; urine, 50-100 µl) were mixed with 6 ml scintillation solvent (Budget-Solve, RPI, Mt. Prospect, IL) to determine total radioactivity. Feces were ground and weighed prior to being homogenized in a 9-fold volume of 0.9% NaCl saline. An aliquot of the homogenate (100 µl) was mixed with solubilizer (TS-2, RPI, Mt. Prospect, IL) and then with scintillation solvent (6 ml) to permit quantitation of total radioactivity.

Following their removal, tissues were immediately blotted on Whatman No. 1 filter paper and weighed prior to being homogenized in 0.9% NaCl saline (3-5 ml per gram of wet weight). The resulting homogenate (100 µl) was mixed with solubilizer (TS-2, RPI, Mt. Prospect, IL) and then with scintillation solvent (6 ml) to determine total radioactivity. The volume of 0.9% NaCl saline added to each tissue sample was recorded. The homogenized tissues/organs were kept frozen at ≤-70°C until the use for further analysis.

### 5. HPLC Analysis

The radioactivity in urine was analyzed by paired-ion HPLC using a modification of the method described essentially by Sands et al. (*Mol. Pharm.* (1994) 45:932-943). Urine samples were centrifuged and passed through a 0.2-µm Acro filter (Gelman, Ann Arbor, MI) prior to analysis. Hybrid oligonucleotide and metabolites in plasma samples were extracted using the above methods in sample preparation for PAGE. A Microsorb MV-C4 column (Rainin Instruments, Woburn, MA) was employed in HPLC using a Hewlett Packard 1050 HPLC with a quaternary pump for gradient making. Mobile phase included two buffers; Buffer A was 5 mM-A reagent (Waters Co., Bedford, MA) in water and Buffer B was 4:1 (v/v) Acetonitrile (Fisher)/water. The column was eluted at a flow rate of 1.5 ml/min, using the following gradient: (1) 0-4 min, 0% buffer B; (2) 4-15 min 0-35% Buffer B; and (3) 15-70 min 35%-80% Buffer B. The column was equilibrated with Buffer A for at least 30 min prior to the next run. By using a RediFrac fraction collector (Pharmacia LKB Biotechnology, Piscataway, NJ), 1-min fractions (1.5 ml) were collected and mixed with 5 ml scintillation solvent to determine radioactivity in each fraction.

### 6. Gel electrophoresis.

Polyacrylamide gel electrophoresis (PAGE) of the extracted oligonucleotides was carried out using methods previously described (Agrawal et al. (1995) *Biochem. Pharmacol.* 50:571-576; Zhang et al. (1995) *Biochem. Pharmacol.* 49:929-939; Zhang et al. (1995) *Biochem. Pharmacol.* 50:571-576; and Zhang et al. (1996) *J. Pharm. Exp. Ther.* 278:971-979). Plasma and tissue homogenates were incubated with proteinase K (2 mg/ml) in extraction buffer (0.5% SDS/10 mM NaCl/20 mM Tris-HCl, pH 7.6/10 mM EDTA) for 1 hr at 60°C. The samples were then extracted twice with phenol/chloroform (1:1, v/v) and once with chloroform. After ethanol precipitation, the extracts were analyzed by electrophoresis in 20% polyacrylamide gels containing 7 M urea. Urine samples were filtered, desalted, and then analyzed by PAGE. The gels were fixed in 10% acetic acid/10% methanol solution and then dried before autoradiography.

## Claims

1. A pharmaceutical formulation comprising an oligonucleotide of about 12 to 50 nucleotides having non-phosphodiester internucleotide linkages and being complementary to a targeted gene, for colorectal administration to a mammal, wherein the oligonucleotide has 3' and 5' termini and further comprises at least one 2'-substituted ribonucleotide at the 5' terminus, at the 3' terminus, or at the 5' terminus and the 3' terminus.

2. The pharmaceutical formulation according to claim 1, wherein the oligonucleotide comprises at least one methylphosphonate deoxynucleotide.

3. The pharmaceutical formulation according to claim 2, wherein all of the nucleotides in the oligonucleotide are 2'-substituted ribonucleotides.

4. The pharmaceutical formulation according to claim 1 or 3, wherein the 2'-substituted ribonucleotide is a 2'-O-alkyl-ribonucleotide.

5. The pharmaceutical formulation according to claim 1, wherein the oligonucleotide comprises at least one deoxyribonucleotide.

6. The pharmaceutical formulation according to claim 5, wherein the oligonucleotide comprises a region of at least four contiguous deoxyribonucleotides capable of activating RNase H activity.

7. The pharmaceutical formulation according to claim 1, wherein the oligonucleotide comprises an internucleotide linkage selected from the group consisting of alkylphosphonates, phosphorothioates, phosphorodithioates, alkylphosphonothioates, phosphoramidates, phosphoramidites, phosphate esters, carbamates, carbonates, phosphate triesters, acetamidate, and carboxymethyl esters.

8. The pharmaceutical formulation according to claim 7, wherein all of the nucleotides are linked via phosphorothioate or phorphorodithioate internucleotide linkages.

9. The pharmaceutical formulation according to any one of claims 1-8, wherein the oligonucleotide comprises modified nucleic acids, bases or sugars other than those found in nature.

10. The pharmaceutical formulation according to any one of claims 1-9, wherein the oligonucleotide further comprises a substituent selected from the group consisting of substitution with diamine, cholestryl, other lipophilic groups, nuclease resistance-conferring bulky substituents at at least one end; caps; substitutions in one non-bridging oxygen per nucleotide; and partial oxidation.

11. The pharmaceutical formulation according to any one of claims 1-10, wherein the oligonucleotide is complementary to a gene of a virus, a pathogenic organism, or a cellular gene.

12. The pharmaceutical formulation according to claim 11, wherein the oligonucleotide is complementary to a gene of a virus involved in a disease selected from the group consisting of AIDS, oral and genital herpes, papilloma warts, influenza, foot and mouth disease, yellow fever, chicken pox, shingles, adult T-cell leukemia, Burkitt's lymphoma, nasopharyngeal carcinoma, and hepatitis.

13. The pharmaceutical formulation according to any one of claims 1-10, wherein the oligonucleotide is complementary to a gene encoding a protein associated with Alzheimer's disease.

14. The pharmaceutical formulation according to any one of claims 1-10, wherein the oligonucleotide is complementary to a gene encoding a protein in a parasite causing a parasitic disease selected from the group consisting of amebiasis, Chagas' disease, toxoplasmosis, pneumocytosis, giardiasis, cryptoporidiosis, trichomoniasis, malaria, ascariasis, filariasis, trichinosis, and schistosomiasis infections.

15. A pharmaceutical formulation according to claim 11 wherein the oligonucleotide is complementary to a HIV gene and comprises about 15 to 26 nucleotides linked by phosphorothioate internucleotide linkages, at least one of the nucleotides at the 3' terminus being a 2'-substituted ribonucleotide or a methylphosphonate deoxynucleotide, and at least four nucleotides being contiguous deoxyribonucleotides.

16. The pharmaceutical formulation according to claim 2, wherein the oligonucleotide has 3' and 5' termini and at least one methylphosphonate deoxynucleotide is at the 3' terminus and the 5' terminus.

17. The pharmaceutical formulation according to claim 16, wherein the oligonucleotide has at least two methylphosphonate deoxynucleotides at the 3' terminus and at the 5' terminus.

18. The pharmaceutical formulation according to claim 17, wherein the oligonucleotide further comprises phosphorothioate internucleotide linkages.

19. The use of an oligonucleotide of about 15 to 25 nucleotides and comprising non-phosphodiester internucleotide linkages for the manufacture of a pharmaceutical formulation for colorectal administration for the treatment of virus infections, infections by pathogenic organisms, or diseases or disorders resulting from abnormal gene expression or from the expression of an abnormal gene product wherein the oligonucleotide has 3' and 5' termini and further comprises at least one 2'-substituted ribonucleotide at the 5' terminus, at the 3' terminus, or at the 5' terminus and the 3' terminus.

20. The use according to claim 19, wherein the oligonucleotide comprises at least one methylphosphonate deoxynucleotide at the 3' terminus, the 5' terminus, or the 3' terminus and the 5' terminus.

21. The use according to claim 19, wherein the oligonucleotide comprises at least two 2'-O-methyl-ribonucleotides or methylphosphonate deoxynucleotides at each terminus.

22. The use according to claim 19, wherein the oligonucleotide comprises at least two 2'-O-methyl-ribonucleotides at each terminus and further comprises phosphorothioate internucleotide linkages.

23. The use according to claim 19, wherein the oligonucleotide comprises at least two methylphosphonate deoxynucleotides at each terminus and further comprises phosphorothioate internucleotide linkages.

24. The use according to claim 19, wherein the oligonucleotide comprises four methylphosphonate deoxynucleotides at each terminus.

25. The use according to claim 20, wherein the oligonucleotide comprises at least two 2'-O-methyl-ribonucleotides or methylphosphonate deoxynucleotides at each terminus.

26. The use according to claim 20, wherein the oligonucleotide comprises at least two 2'-O-methyl-ribonucleotides at each terminus and further comprises phosphorothioate internucleotide linkages.

27. The use according to claim 20, wherein the oligonucleotide comprises at least two methylphosphonate deoxynucleotides at each terminus and further comprises phosphorothioate internucleotide linkages.

28. The use according to claim 20, wherein the oligonucleotide comprises four methylphosphonate deoxynucleotides at each terminus.

29. The use according to claim 19, wherein the oligonucleotide comprises phosphorothioate internucleotide linkages.

30. The use of an oligonucleotide according to any one of claims 1 to 29 for the manufacture of a pharmaceutical composition for colorectal administration to a mammal.

31. The use according to claim 19, wherein the oligonucleotide comprises at least one 2'-O-methyl ribonucleotide at the 5' terminus, at the 3' terminus or at the 3' terminus and the 5' terminus.

## Patentansprüche

1. Eine pharmazeutische Formulierung umfassend ein Oligonukleotid von ungefähr 12 bis 50 Nukleotiden, das Nicht-Phosphodiester Internukleotid Verbindungen besitzt und komplementär zu einem angezielten Gen ist, für die kolorektale Verabreichung an einen Säuger, wobei das Oligonukleotid 3' und 5' Termini besitzt, und ferner wenigstens ein 2'-substituiertes Ribonukleotid an dem 5' Terminus, an dem 3' Terminus oder an dem 5' Terminus und dem 3' Terminus umfasst.

2. Die pharmazeutische Formulierung gemäß Anspruch 1, wobei das Oligonukleotid wenigstens ein Methlyphosphonat Deoxynukleotid umfasst.

3. Die pharmazeutische Formulierung gemäß Anspruch 2, wobei alle Nukleotide in dem Oligonukleotid 2'-substituierte Ribonukleotide sind.

4. Die pharmazeutische Formulierung gemäß Anspruch 1 oder 3, wobei das 2'-substituierte Ribonukleotid ein 2'-O-Alkyl-Ribonukleotid ist.

5. Die pharmazeutische Formulierung gemäß Anspruch 1, wobei das Oligonukleotid wenigstens ein Deoxyribonukleotid umfasst.

6. Die pharmazeutische Formulierung gemäß Anspruch 5, wobei das Oligonukleotid einen Bereich von wenigstens vier zusammenhängenden Deoxyribonukleotiden umfasst, die fähig sind RNase H Aktivität zu aktivieren.

7. Die pharmazeutische Formulierung gemäß Anspruch 1, wobei das Oligonukleotid eine Internukleotid Verbindung umfasst ausgewählt aus der Gruppe bestehend aus Alkylphosphonaten, Phosphorothioaten, Phosphorodithioaten, Alkylphosphonothioaten, Phosphoramidaten, Phosphoramiditen, Phosphatestern, Carbamaten, Carbonaten, Phosphattriestern, Acetamidat, und Carboxymethylestern.

8. Die pharmazeutische Formulierung gemäß Anspruch 7, wobei alle Nukleotide über Phosphorothioat- oder Phosphorodithioat-Internukleotid Verbindungen verbunden sind.

9. Die pharmazeutische Formulierung gemäß einem der Ansprüche 1-8, wobei das Oligonukleotid andere modifizierte Nukleinsäuren, Basen oder Zucker umfasst, als diejenigen, die in der Natur vorkommen.

10. Die pharmazeutische Formulierung gemäß einem der Ansprüche 1-9, wobei das Oligonukleotid ferner einen Substituenten umfasst ausgewählt aus der Gruppe bestehend aus Substitution mit Diamin, Cholesteryl, anderen lipophilen Gruppen, Nukleaseresistenz verleihenden sperrigen Substituenten an wenigstem einem Ende; Kappen; Substitutionen in einem nicht-überbrückenden Sauerstoff pro Nukleotid; und teilweise Oxidation.

11. Die pharmazeutische Formulierung gemäß einem der Ansprüche 1-10, wobei das Oligonukleotid komplementär ist zu einem Gen eines Virus, eines pathogenen Organismus, oder zu einem zellulären Gen.

12. Die pharmazeutische Formulierung gemäß Anspruch 11, wobei das Oligonukleotid komplementär zu einem Gen eines Virus ist, der involviert ist bei einer Krankheit ausgewählt aus der Gruppe bestehend aus AIDS, orale oder genitale Herpes, Papilloma Warzen, Influenza, Maul und Klauen Seuche, Gelbfieber, Windpocken, Gürtelrose, adulte T-Zell Leukämie, Burkitt Lymphoma, Nasopharyngeales Karzinom und Hepatitis.

13. Die pharmazeutische Formulierung gemäß einem der Ansprüche 1-10, wobei das Oligonukleotid komplementär ist zu einem Gen, das für ein Protein kodiert, das mit der Alzheimer Krankheit assoziiert ist.

14. Die pharmazeutische Formulierung gemäß einem der Ansprüche 1-10, wobei das Oligonukleotid komplementär ist zu einem Gen, das für Protein in einem Parasiten kodiert, der eine Parasitenkrankheit verursacht ausgewählt aus der Gruppe bestehend aus Amebiasis, Chagaskrankheit, Toxoplasmosis, Pneumocytosis, Lambliasis, Kryptoporidiosis, Trichomoniasis, Malaria, Askaridiasis, Filariasis, Trichinose und Schistosomiasis Infektionen.

15. Eine pharmazeutische Formulierung gemäß Anspruch 11, wobei das Oligonukleotid komplementär ist zu einem HIV Gen und ungefähr 15 bis 26 Nukleotide umfasst verbunden durch Phosphorothioat Verbindungen, wobei wenigstes eines der Nukleotide an dem 3' Terminus ein 2' substituiertes Ribonukleotid oder ein Methylphosphonat Deoxynukleotid ist, und wobei wenigstens vier Nukleotide zusammenhängende Deoxyribonukleotide sind.

16. Die pharmazeutische Formulierung gemäß Anspruch 2, wobei das Oligonukleotid 3' und 5' Termini besitzt und wenigstens ein Methylphosphonat Deoxynukleotid an dem 3' Terminus und dem 5' Terminus ist.

17. Die pharmazeutische Formulierung gemäß Anspruch 16, wobei das Oligonukleotid wenigstens zwei Methylphosphonat Deoxynukleotide an dem 3' Terminus und dem 5' Terminus besitzt.

18. Die pharmazeutische Formulierung gemäß Anspruch 17, wobei das Oligonukleotid ferner Phosphorothioat Internukleotid Verbindungen umfasst.

19. Die Verwendung eines Oligonukleotids von ungefähr 15 bis 25 Nukleotiden und umfassend Nicht-Phosphodiester Internukleotid Verbindungen für die Herstellung einer pharmazeutischen Formulierung für kolorektale Verabreichung für die Behandlung von Virusinfektionen, Infektionen durch pathogene Organismen, oder Krankheiten oder Funktionsstörungen, die aus abnormaler Genexpression oder aus der Expression eines abnormalen Genproduktes resultieren, wobei das Oligonukleotid 3' und 5' Termini besitzt, und ferner wenigstens ein 2'-substituiertes Ribonukleotid an dem 5' Terminus, an dem 3' Terminus oder an dem 5' Terminus und dem 3' Terminus umfasst.

20. Die Verwendung gemäß Anspruch 19, wobei das Oligonukleotid wenigstens ein Methylphosphonat Deoxynukleotid an dem 3' Terminus, dem 5' Terminus oder dem 3' Terminus und dem 5' Terminus umfasst.

21. Die Verwendung gemäß Anspruch 19, wobei das Oligonukleotid wenigstens zwei 2'-O-Methyl-Ribonukleotide oder Methylphosphonat Deoxynukleotide an jedem Terminus umfasst.

22. Die Verwendung gemäß Anspruch 19, wobei das Oligonukleotid wenigstens zwei 2'-O-Methyl-Ribonukleotide an jedem Terminus umfasst und ferner Phosphorothioat Internukleotid Verbindungen umfasst.

23. Die Verwendung gemäß Anspruch 19, wobei das Oligonukleotid wenigstens zwei Methylphosphonat Deoxynukleotide an jedem Terminus umfasst und ferner Phosphorothioat Internukleotid Verbindungen umfasst.

24. Die Verwendung gemäß Anspruch 19, wobei das Oligonukleotid wenigstens vier Methylphosphonat Deoxynukleotide an jedem Terminus umfasst.

25. Die Verwendung gemäß Anspruch 20, wobei das Oligonukleotid wenigstens zwei 2'-O-Methyl-Ribonukleotide oder Methylphosphonat Deoxynukleotide an jedem Terminus umfasst.

26. Die Verwendung gemäß Anspruch 20, wobei das Oligonukleotid wenigstens zwei 2'-O-Methyl-Ribonukleotide an jedem Terminus umfasst und ferner Phosphorothioat Internukleotid Verbindungen umfasst.

27. Die Verwendung gemäß Anspruch 20, wobei das Oligonukleotid wenigstens zwei Methylphosphonat Deoxynukleotide an jedem Terminus umfasst und ferner Phosphorothioat Internukleotid Verbindungen umfasst.

28. Die Verwendung gemäß Anspruch 20, wobei das Oligonukleotid wenigstens vier Methylphosphonat Deoxynukleotide an jedem Terminus umfasst.

29. Die Verwendung gemäß Anspruch 19, wobei das Oligonukleotid Phosphorothioat Internukleotid Verbindungen umfasst.

30. Die Verwendung eines Oligonukleotids gemäß einem der Ansprüche 1 bis 29 für die Herstellung einer pharmazeutischen Zusammensetzung für kolorektale Verabreichung an einen Säuger.

31. Die Verwendung gemäß Anspruch 19, wobei das Oligonukleotid wenigstens ein 2'-O-Methyl-Ribonukleotid an dem 5' Terminus, an dem 3' Terminus oder dem 3' Terminus und dem 5' Terminus umfasst.

## Revendications

1. Formulation pharmaceutique comprenant un oligonucléotide d'environ 12 à 50 nucléotides ayant des liaisons internucléotidiques non-phosphodiesters et étant complémentaire d'un gène ciblé, pour administration colorectale à un mammifère, dans laquelle l'oligonucléotide a des extrémités 3' et 5' et comprend en outre au moins un ribonucléotide 2'-substitué à l'extrémité 5', à l'extrémité 3' ou à l'extrémité 5' et à l'extrémité 3'.

2. Formulation pharmaceutique selon la revendication 1, dans laquelle l'oligonucléotide comprend au moins un désoxynucléotide méthylphosphonate.

3. Formulation pharmaceutique selon la revendication 2, dans laquelle tous les nucléotides dans l'oligonucléotide sont des ribonucléotides 2'-substitués.

4. Formulation pharmaceutique selon la revendication 1 ou 3, dans laquelle le ribonucléotide 2-substitué est un 2'-O-alkyl-ribonucléotide.

5. Formulation pharmaceutique selon la revendication 1, dans laquelle l'oligonucléotide comprend au moins un désoxyribonucléotide.

6. Formulation pharmaceutique selon la revendication 5, dans laquelle l'oligonucléotide comprend une région d'au moins quatre désoxyribonucléotides contigus capables d'activer une activité RNase H.

7. Formulation pharmaceutique selon la revendication 1, dans laquelle l'oligonucléotide comprend une liaison internucléotidique choisie dans l'ensemble constitué d'alkylphosphonates, de phosphorothioates, de phosphorodithioates, d'alkylphosphonothioates, de phosphoramidates, de phosphoramidites, de phosphate esters, de carbamates, de carbonates, de phosphate triesters, d'acétamidate et de carboxyméthylesters.

8. Formulation pharmaceutique selon la revendication 7, dans laquelle tous les nucléotides sont liés par des liaisons internucléotidiques phosphorothioates ou phosphophorodithioates.

9. Formulation pharmaceutique selon l'une quelconque des revendications 1-8, dans laquelle l'oligonucléotide comprend des acides nucléiques, des bases ou des sucres modifiés différents de ceux que l'on trouve dans la nature.

10. Formulation pharmaceutique selon l'une quelconque des revendications 1-9, dans laquelle l'oligonucléotide comprend en outre un substituant choisi dans l'ensemble constitué d'une substitution par un groupe diamine, cholestryle, d'autres groupes lipophiles, des substituants volumineux conférant une résistance aux nucléases à au moins une extrémité ; des coiffes ; des substitutions dans un oxygène ne formant pas un pont par nucléotide ; et une oxydation partielle.

11. Formulation pharmaceutique selon l'une quelconque des revendications 1-10, dans laquelle l'oligonucléotide est complémentaire d'un gène d'un virus, d'un organisme pathogène ou d'un gène cellulaire.

12. Formulation pharmaceutique selon la revendication 11, dans laquelle l'oligonucléotide est complémentaire d'un gène d'un virus impliqué dans une maladie choisie dans l'ensemble constitué du SIDA, d'herpès oraux et génitaux, de verrues à papillomes, de la grippe, de la fièvre aphteuse, de la fièvre jaune, de la varicelle, du zona, de la leucémie à cellules T de l'adulte, du lymphome de Burkitt, du carcinome naso-pharyngique, et de l'hépatite.

13. Formulation pharmaceutique selon l'une quelconque des revendications 1-10, dans laquelle l'oligonucléotide est complémentaire d'un gène codant une protéine associée à la maladie d'Alzheimer.

14. Formulation pharmaceutique selon l'une quelconque des revendications 1-10, dans laquelle l'oligonucléotide est complémentaire d'un gène codant une protéine dans un parasite provoquant une maladie parasitaire choisie dans l'ensemble constitué de l'amibiase, la maladie de Chagas, la toxoplasmose, la pneumocytose, la giardiase, la cryptoporidiose, la trichomonase, la malaria, l'ascaridiase, la filariose, la trichinose, et des infections de bilharziose.

15. Formulation pharmaceutique selon la revendication 11, dans laquelle l'oligonucléotide est complémentaire d'un gène du VIH et comprend environ 15 à 26 nucléotides liés par des liaisons internucléotidiques phosphorothioate, au moins un des nucléotides à l'extrémité 3' étant un ribonucléotide 2'-substitué ou un désoxynucléotide méthylphosphonate et au moins quatre nucléotides étant des désoxyribonucléotides contigus.

16. Formulation pharmaceutique selon la revendication 2, dans laquelle l'oligonucléotide a des extrémités 3' et 5' et au moins un désoxynucléotide méthylphosphonate se trouve à l'extrémité 3' et à l'extrémité 5'.

17. Formulation pharmaceutique selon la revendication 16, dans laquelle l'oligonucléotide a au moins deux méthylphosphonate désoxynucléotides à l'extrémité 3' et à l'extrémité 5'.

18. Formulation pharmaceutique selon la revendication 17, dans laquelle l'oligonucléotide comprend en outre des liaisons internucléotidiques phosphorothioates.

19. Utilisation d'un oligonucléotide d'environ 15 à 25 nucléotides et comprenant des liaisons internucléotidiques non-phosphodiesters pour la préparation d'une formulation pharmaceutique pour administration colorectale pour le traitement d'infections virales, d'infections par des organismes pathogènes ou de maladies ou d'affections provoquées par une expression anormale de gène ou par l'expression d'un produit de gène anormal, dans laquelle l'oligonucléotide a des extrémités 3' et 5' et comprend en outre au moins un ribonucléotide 2'-substitué à l'extrémité 5', à l'extrémité 3' ou à l'extrémité 5' et à l'extrémité 3'.

20. Utilisation selon la revendication 19, dans laquelle l'oligonucléotide comprend au moins un désoxynucléotide méthylphosphonate à l'extrémité 3', à l'extrémité 5' ou à l'extrémité 3' et à l'extrémité 5'.

21. Utilisation selon la revendication 19, dans laquelle l'oligonucléotide comprend au moins deux 2'-O-méthyl-ribonucléotides ou désoxynucléotide méthylphosphonate à chaque extrémité.

22. Utilisation selon la revendication 19, dans laquelle l'oligonucléotide comprend au moins deux 2'-O-méthyl-ribonucléotides à chaque extrémité et comprend en outre des liaisons internucléotidiques phosphorothioates.

23. Utilisation selon la revendication 19, dans laquelle l'oligonucléotide comprend au moins deux méthylphosphonate désoxynucléotides à chaque extrémité et comprend en outre des liaisons internucléotidiques phosphorothioates.

24. Utilisation selon la revendication 19, dans laquelle l'oligonucléotide comprend quatre désoxynucléotides méthylphosphonate à chaque extrémité.

25. Utilisation selon la revendication 20, dans laquelle l'oligonucléotide comprend au moins deux 2'-O-méthyl-ribonucléotides ou désoxynucléotides méthylphosphonate à chaque extrémité.

26. Utilisation selon la revendication 20, dans laquelle l'oligonucléotide comprend au moins deux 2'-O-méthyl-ribonucléotides à chaque extrémité et comprend en outre des liaisons internucléotidiques phosphorothioates.

27. Utilisation selon la revendication 20, dans laquelle l'oligonucléotide comprend au moins deux désoxynucléotides méthylphosphonate à chaque extrémité et comprend en outre des liaisons internucléotidiques phosphorothioates.

28. Utilisation selon la revendication 20, dans laquelle l'oligonucléotide comprend quatre désoxynucléotides méthylphosphonate à chaque extrémité.

29. Utilisation selon la revendication 19, dans laquelle l'oligonucléotide comprend des liaisons internucléotidiques phosphorothioates.

30. Utilisation d'un oligonucléotide selon l'une quelconque des revendications 1 à 29 pour la fabrication d'une composition pharmaceutique pour administration colorectale à un mammifère.

31. Utilisation selon la revendication 19, dans laquelle l'oligonucléotide comprend au moins un 2'-O-méthyl-ribonucléotide à l'extrémité 5', à l'extrémité 3' ou à l'extrémité 3' et à l'extrémité 5'.
